Europäisches Patentamt

European Patent Office

Office européen des brevets

(19)

(11) Publication number: **0 180 564 B1**

# EUROPEAN PATENT SPECIFICATION

(45) Date of publication of patent specification: **17.07.91**

(51) Int. Cl.⁵: **A61K 39/385**, A61K 39/44, A61K 39/39, A61K 45/05

(21) Application number: **85850326.1**

(22) Date of filing: **16.10.85**

(54) **Immunogenic complex, a method for producing the same, and the use thereof as an immune stimulant, vaccines and reagents.**

(30) Priority: **01.11.84 SE 8405493**

(43) Date of publication of application:
**07.05.86 Bulletin 86/19**

(45) Publication of the grant of the patent:
**17.07.91 Bulletin 91/29**

(84) Designated Contracting States:
**AT BE CH DE FR GB IT LI LU NL SE**

(56) References cited:
**EP-A- 0 037 931      EP-A- 0 109 942
EP-A- 0 117 783      EP-A- 0 142 192
WO-A-84/03506        GB-A- 2 104 527
US-A- 4 157 390**

**CHEMICAL ABSTRACTS, vol. 96, no. 21, 24th
May 1982, page 285, abstract no. 176474d,
Columbus, Ohio, US; B. MOREIN et al.:
"Protein micelles and virosomes from the
surface glycoproteins of parainfluenza 3
virus"**

(73) Proprietor: **Morein, Bror
Ollonstigen 3 Vreta
S-75590 Uppsala(SE)**

(72) Inventor: **Morein, Bror
Ollonstigen 3 Vreta
S-75590 Uppsala(SE)**

(74) Representative: **Fagerlin, Heléne et al
H. ALBIHNS PATENTBYRA AB P.O. Box 3137
S-103 62 Stockholm(SE)**

EP 0 180 564 B1

**Description**

The present invention relates to an immunogenic complex of an immunogenic carrier coupled to one or more molecules which it is desired to render immunogenic or to amplify the immunogenicity thereof, in order to induce the formation of antibodies for analysis purposes, diagnose sickness and disease, biological target searching or the manufacture of vaccine.

Vaccine has traditionally consisted of whole micro-organisms which have been rendered innocous or non-pathogenic, by killing or attenuating the same. For example, viruses have been attenuated by cultivation in a foreign host or host cell, or have been cultivated under other special conditions.

The next generation of vaccine is based on the specific micro-organism components responsible for stimulating the immunity providing the protection against infection. Such components can be given improved immunogenicity, by arranging the same in physically defined multimerforms, such as protein micelles, in lipid vesiclar (liposomes), provided that there is a hydrophobic region present (Morein et al, 1978, Effective subunit vaccines against enveloped animal viruses; Nature, 276, 715-718). A still better immunogenic effect has been obtained, by incorporated hydrophobic proteins or peptides in a complex, (EPC-application 83850273.0) which has been given the name iscom.

It is expected that third and fourth vaccine generations will consist of peptides and proteins produced by recombinant-DNA-techniques, or synthetically produced peptides, or carbohydrates. The latter may be produced synthetically or may be produced in pure form from, for example, biological material optionally bound to peptide-protein or lipid. Many such products are produced and are potential vaccines. Hitherto, all efforts to render such products sufficiently immunogenic with the aid of general methods have been unsuccessful, unless the products are coupled to a carrier structure, such as bovine serumalbumine or to KLH (Keyhole Limpet Haemocyanine) for example, and mixed with an adjuvant, for example oil adjuvant. Such products, however, are unacceptable as vaccines, inter alia because the carrier structure cannot be accepted and because of pronounced side-effects. In addition, in the majority of cases, the requisite antigen dosage is from 100 to 1000 times greater than that required when corresponding antigen structures are used for immunizing purposes and are present in the micro-organism; at times the antigen dosage required can be greater than the afore-said 1000 times.

When used in a conventional manner, known adjuvants are only effective when used in such high dosages as to produce unacceptable side effects. In an attempt to avoid this, an adjuvant designated muranyldipeptide (MDP) has been covalently bound to an antigen (a peptide) and thereby achieved an adjuvant effect with MPD in low dosages. Arnon, R., Sela, M., Parant, M. et Chedid, L., 1980, Proc.na. Acad. Sc. USA 77, 6769-6772. This type of conjugate is relatively difficult or expensive to produce, and has hitherto been restricted to the experimental stage.

It has now been found that proteins and peptides, particularly those which have been prepared synthetically, carbohydrates, glycolipides and other small molecules, for example biotin, and particularly those which are not sufficiently immunogenic, can be made immunogenic, by coupling the same to an immunogenic carrier complex, a so-called iscom, in accordance with EPC-application 83850273.0.

An iscom is a complex between a glycoside (adjuvant) and hydrophobic antigenic proteins or peptides. These proteins or peptides are such that can be accepted for use in the preparation of vaccines. The adjuvant (the glycoside) is able to work in the iscom in concentrations far below those required when adjuvants are used in a conventional manner, and mixed with the antigen. The problem of side-effects caused by the adjuvant is avoided herewith.

In accordance with the invention an iscom product containing an immunogenic peptide or an immunogenic protein, and thus stimulating immune response, inter alia antibody production thereagainst, can now be coupled to the aforesaid molecules. The primary iscom forms the carrier structure to which the molecules are coupled. In this way antibodies are obtained both against the peptide or the protein present in the primary iscom, and against the molecules coupled thereto.

The primary iscom for binding a peptide which e.g. is going to be used in a vaccine should preferably include an envelope protein which in itself is useful as a vaccine. Further several peptides representing determinants (epitopes) from different microorganisms can be linked to the primary iscom which in itself is a vaccine against one or several microorganisms or viruses. In the described way multivalent vaccines can be prepared.

The new complex has the same morphological structure under electron-microscopy as the carrier structure, i.e. the primary iscom. A change in morphology is observed, however, when large molecules are coupled to the primary iscom. Mice were immunized with the complex according to the invention in dosages of approximately 1-10 µg and gave rise to immune response in the absence of any noticeable side-effects. It may be necessary to increase or lower the dosage, and to immunize more than once.

EP 0 180 564 B1

The molecules coupled to the primary iscom may be peptides, proteins, carbohydrates or other molecules which, similar to the peptides or proteins in the primary iscom, have been recovered from micro-organisms (see below) or represent antigenic determinants of the micro-organisms. If large quantities of iscom are available, it may be more practical to couple to a prepared iscom that peptide or that protein against which antibodies are desired, rather than prepare a new iscom.

Those proteins and peptides complex-bound within a primary iscom must be hydrophobic (cf EPC-application 83850273.0). Those molecules coupled to the primary iscom according to the present invention need not be hydrophobic, but need only have some kind of coupling molecules, examples of which are given hereinafter.

Certain kinds of micro-organisms exist which can not be cultivated for vaccine on a large scale. Malaria is one example. Antigenic amino acid sequences (peptides) have been synthetized which correspond to antigenic determinants on malaria proteins, which are considered to afford protective immunity. These peptides, however, have not been found sufficiently immunogenic. These peptides often lack suitable hydrophobicity for the formation of iscoms. Although it is possible to couple a hydrophobic molecule to the peptides and prepare iscom therefrom, it may be more suitable, inter alia from a technical or immunogenity aspect, to couple the peptides to a primary iscom which has already been prepared. This also enables a combined vaccine to be produced, for both malaria and, for example, poliomyelitis (complex-bound poliopeptide within the primary iscom).

The peptides coupled to primary iscom are preferably synthetic or purified from micro-organisms and are often analyzed to ensure that they contain antigenic determinants. A classic size for determinant epitope is 1-4 amino acids. They preferably contain up to 40 amino acids, suitably from 10-25 amino acids. Peptides possessing more than 25 amino acids are difficult to synthesize and tend to fold and hide the antigen determinants.

If the peptides are short and contain less than about 10 amino acids, or if they contain hydrophobic groups which tend to fold or bend into hydrophobic parts in the primary iscom, it may be suitable to move them out from the primary iscom, by extending them with so-called spacers, which are aliphatic chains having 2, 3, 4, 5, 6, 7, 8, 9, 10, 11 or 12 carbon atoms, suitably 6, 7, 8 carbon atoms having 6, 7, 8, 9, 10, 11, 12, 13, 14, 15, 16, 17, 18, 19, 20, 21, 22, 23, 24, 25, 26 OH-groups, which renders the aliphatic chain hydrophilic, and a functional coupling group (such as those recited on p. 7) at each end of the chain, for example an $NH_2$-, COOH-, SH- or OH-group, such as glucose amines, or a peptide comprising 1, 2, 3, 4, 5, 6, 7, 8, 9, 10, 11, 12, 13, 14, 15, 16, 17, 18, 19, 20 amino acids, preferably hydrophilic amino acids, for example glycine or proline, such as polyglycine or polyproline. The spacer is preferably first coupled to the primary iscom and then to the peptide through the terminal functional groups, using one of the following coupling methods herefor.

Examples of molecules which can be coupled to the carrier molecule include cyclic or linear proteins and peptides derived from those micro-organisms hereinafter recited with respect to the preparation of iscom; linear or cyclic synthetic peptides or peptides prepared with the aid of hybrid-DNA-techniques with amino acids sequences, for example representative for Malaria, peptides derived from Polio-virus, particularly 277-300 peptides derived from Hepatite-B-virus, particularly peptides in the regions 32-74, 110-156; and particularly peptides containing the amino acid sequences 144-145; Rabies virus, particularly peptides in the regions 1-50, 290-320, and particularly in the region 100-175; Influenza-virus peptides containing the amino acid sequence 140-146, 187-196 or selected within the region Cys 52-Cys 278 or 207-220; foot-and-mouth disease virus, particularly the foot-and-mouth disease virus peptides 141-160, 144-160, 146-154, 144-150, 142-158; growth factors for T-cells (Interleukin II), preferably peptides having the amino acid sequences 79-92, 139-153C, 111-125 and 18-32C, peptides from Epstein Barr Virus, particularly the sequences A: Asp, Val, Gly, Gly, Lys, Lys, His, Gln, Lev, Asp, Cys, Leu, Leu, B: His, His, Ala, Glu, Asn, Gln, Asn, Pro, Cys, Leu, Leu, C: Ala, Trp, Pro, Asn, Asn, Thr, Glu, Thr, Asp, Phe, Lys, Cys, Leu, Leu; antigenic determinals from HTLV 1, 2 or 3 virus; peptides in blood group substances.

In accordance with the invention, it is also possible to couple to primary iscom peptides, polypeptides and long-chain proteins against which it is desired to produce antibodies, and in particular hormones found within the groups steroid hormones, peptide hormones and prostaglandines, of which the following can be mentioned by way of example: tyrotropine, adenocorticotrophin, luteinizing hormone, gonadotrophin-release hormone (LHRH), follicle-stimulating hormone, prolactin, growth hormone, oxytocin, vasopessin, paratyroidea hormone, calcitonin, insulin, glucagon. It is also desired to prepare antibodies against enzymes which can be coupled in accordance with the invention. Lysosyme and peroxidase can be mentioned by way of example in this connection. These enzymes may include more than 40 amino acids.

It is also possible to couple to the iscom carbohydrates and carbohydrate-containing structures, such as lipopoly saccharides, polysaccharides derived from encapsulated micro-organisms, such as E.coli; and in

3

particular the K-antigenes 1-13, Haemophilus influenza, meningococcus, the oligosaccharide portion of glycoproteins, in blood group substances, in gangliosides, such as GM1, and gliomgangliosides. A practical advantage may be afforded in producing antibodies against blood group substances, for blood group analyses. The gliomgangliosides are formed by changes occurring in gangliosides present in brain tumours, and it might be possible to use antibodies produced against these gliomgangliosides to diagnose brain tumours.

Other examples of substances which can be coupled to the carrier are lipoproteins, other glycoproteins, and biotin.

These molecules can be coupled to the primary iscom via functional coupling groups already found in or coupled to the molecules and functional groups in the primary iscom, e.g. in amino acids in the proteins or peptides or HO-, CHO- or HOOC-groups in the glycoside. Known coupling reactions are applied in this respect. The most suitable coupling reactions in this regard are:

| Functional group on the molecule to be coupled | Activating reagent | Functional group in the primary iscom |
|---|---|---|
| $-NH_2$ ($\alpha$ or $\varepsilon$) | Glutaraldehyde | $-NH_2$ ($-SH$) |
| | Carbodiimides | $-CO_2H$ |
| | Diimidoesters | $-NH_2$ |
| | Diisocyanates | $-NH_2$ ($-OH$) |
| | $MeO-Cl_2$-triazine | $-OH$, $-SH$, $-NH_2$ |
| | Arylhalogenides | $-NH_2$, $-OH$, $-SH$ |
| $-CO_2H$ | Carbodiimides | $-NH_2$ ($-OH$) |
| $-SH$ | $MeO-Cl_2$-triazine | $-OH$, $-SH$, $-NH_2$ |
| | Alkylhalogenides | $-SH$, ($-NH_2$, His) |
| | Iodoacetic acid+carbo diimide | $-NH_2$ |
| | Maleimides | $-SH$ |
| $-OH$ (Tyr) | $MeO-Cl_2$-triazine | $-OH$, $-SH$, $-NH_2$ |
| | Arylhalogenides | $-NH_2$, $-OH$, $-SH$ |
| Aromates with Tyr, His | Bis-diazonium compounds | Aromatic Tyr, His, Lys |
| CHO | pH adjustment over 8 | $-NH_2$ |
| Vicinal | perjodate forms CHO | $-NH_2$ |
| OH | pH adjustment over 8 | |
| CHO | Hydrazine | |
| OH | pH adjustment to 9-10 | $-NH_2$ |
| $NH_2$ | as above | CHO, OH |

Several coupling groups and methods are found described in Journal of Immunological Methods, 59 (1983), 129-143, 289-299; Methods in Enzymology, Vol 93, pages 280-333; and Analytical Biochemistry 116, 402-407 (1981).

The novel complex according to the invention is prepared by first producing a carrier, primary iscom, and then coupling thereto those molecules which are to be rendered immunogenic or the immunogenicity of which is to be strengthened.

I. The preparation of iscom (primary iscom)

An immunogenic complex between antigenic proteins or peptides having hydrophobic regions and glycoside is prepared by mixing proteins or peptides having hydrophobic regions with one or more solubilizing agents, whereby a complex is formed between charged monomeric antigenic proteins or peptides and solubilising agent, whereafter the charged monomeric antigenic proteins or peptides are separated from the solubilizing agent in the presence of a glycoside solution which contains one or more glycosides possessing hydrophobic and hydrophilic regions in a concentration equal at least to the critical micellular concentration or, alternatively, are separated from the solubilizing agent and transferred directly to the aforesaid glycoside solution, whereupon a complex is formed between the proteins or peptides and the glycoside, this complex being isolated and purified.

The proteins or peptides with hydrophobic regions that are complexed to hydrophobic regions of the glycosides may be

A) amphiphatic proteins or peptides with hydrophilic and hydrophobic groups derived from or being membrane proteins or membrane peptides from enveloped viruses, bacteria, mycoplasmas, parasites or animal cells, or such proteins or peptides produced by hybrid DNA technique, or molecules produced synthetically,

B) hydrophilic proteins or peptides made amphiphatic by hydrophobic groups being coupled to them. These proteins or peptides may derive from viruses, bacteria, mycoplasmas, parasites, animal cells, or be synthesized or obtained by hybrid DNA technique,

C) amphiphatic proteins or peptides obtained by inaccessible hydrophobic parts of hydrophilic proteins being made accessible by chemical means. These proteins may derive from the micro-organisms or cells mentioned above or obtained by hybrid DNA technique, or be synthesized.

a) Concerning the preparation of membrane proteins or membrane peptides derived from whole cells or viruses, the preparation of the complexes comprises in principle three steps: purification or isolation of animal cells or microorganisms or fragments thereof; solubilizing of hydrophobic proteins and removal of the solubilizing agent while at the same time transferring the desired antigen in complex by means of glycoside in an immunogenic form (immunogenic complex).

Purification and isolation

Viruses with envelope, mycoplasmas, bacteria, parasites and animal cells are concentrated and purified in a known manner (for references see "The Tools of Biochemistry", T G Cooper, John Wiley & Sons (1977) New York, which is incorporated as a reference), for example by centrifuging, ultracentrifuging, electrophoresis and different chromatographic methods such as gel filtration, hydrophobic interaction, affinity chromatography or centrifuging through a sugar gradient or gradient centrifuging through percoll or with hollow fiber dialysis. For bacteria, it can be necessary or more advantageous to first lyze or break down the cell walls (for references see Cota-Robles and Stein, CRC Handbook of Microbiology Vol II (1973) pp 833-844) with e.g. ultrasond or French press treatment for example.

Solubilizing

The purified animal cells or microorganisms or fragments thereof are then mixed with ionic, non-ionic or Zwitterionic detergent, which is used in excess. Typical examples of suitable non-ionic detergents are polyglycol esters and polyglycol ethers with aliphatic or arylaliphatic acids and alcohols. Examples of these are alkylpolyoxyethylene ethers with the general formula $C_nH_{2n+1}(OCH_2CH_2)_xOH$, shortened to $C_nE_x$; alkylphenyl polyoxyethylene ethers containing a phenyl ring between the alkyl group and the polyoxyethylene chain, abbreviated $C_n\phi E_x$, e.g. Triton® X-100 = tert.-$C_8\phi E_{9,6}$ (octylphenol ether of polyethylene oxide), acylpolyoxyethylene esters; acylpolyoxyethylene sorbitane esters, abbreviated $C_n$ sorbitane $E_x$, e.g. Tween®20, Tween®80, $\beta$-D-alkylglucosides, e.g. $\beta$-D-octylglucoside. The glycosides mentioned below can also be used, especially saponin. These are, however, weak detergents and should be used together with other detergents. Typical examples of suitable ionic detergents are gallic acid detergents such as e.g. desoxycholate and cholate. Even conjugated detergents such as e.g. taurodeoxycholate, glycodeoxycholate and glycocholate can be used. Possible Zwitterionic detergents are lysolecitin and synthetic lysophospholipids. Even mixtures of the above-mentioned detergents can be used.

Solubilizing can also be performed with alcohols, organic solvents or small amphiphatic molecules such as heptane-1,2,3-triol, hexane-1,2,3-triol, acetic acid, or mixtures thereof, or with detergents.

The solubilizing agent is used in excess in relation to the amount of lipid and hydrophobic proteins.

EP 0 180 564 B1

Suitably cells or microorganisms and detergent are mixed in the weight ratio 1:3 to 1:10.

Cells or microorganisms and solubilizing agent are mixed in buffered possibly saline solution. The molarity of the saline solution lies between 0.02 and 0.5 M, preferably between 0.05 and 0.25 M. 0.1-0.2 M is preferred. The detergent should act for about 1 hour at room temperature.

Sodium chloride is preferred as a salt, but other salts can also be considered, especially salts with alkali ions, earth alkali ions and ammonium ions and strong mineral acids and organic acids such as acetic acid, trichloroacidic acid, formic acid and oxalic acid. As a buffer, all substances are suitable which buffer in the pH interval 6.5-9. When using cholates and desoxycholates, pH 8-9 is preferred, and when using non-ionic detergents, pH 7.4. When organic acids are used for protein solubilizing, buffer may be omitted.

The preparation of immunogenic complexes

When cells or microorganisms have been solubilized, a mixture of solubilizing agent and cell or microorganism fragments are formed (hereinafter called fragments). Among the fragments there are charged monomeric antigenic proteins with hydrophobic regions in complex with the solubilizing agent. The immunogenic complex (primary iscom) is produced by separating the charged monomeric antigenic proteins from the solubilizing agent in the presence of, or by directly transferring to, one or more glycosides which must have hydrophobic and hydrophilic regions and be present in a concentration of at least the critical micelle concentration. The rest of the fragments are removed before the complex according to the invention is produced, while it is being produced, or afterwards, preferably before.

The carrier complex can be produced either by removing the solubilizing agent, e.g. by dialysis, gel filtration or chromatography from the mixture of solubilizing agent, charged monomeric antigenic proteins, glycoside and possibly other fragments or by separating the charged, monomeric antigenic proteins from said mixture, e.g. by gradient centrifuging, chromatography or electrophoresis. The essential feature is that the monomeric antigenic proteins are separated from the solubilizing agent during the simultaneous presence of the glycoside or after separation are directly transferred to the glycoside, of which the micelle form should be present. When the monomeric antigenic proteins are separated from the solubilizing agent so that they can come directly into contact with the glycoside, the special complex according to the invention is formed. It is assumed that the micelle form of the glycoside is the base for forming the complex and that this is formed by attraction between hydrophobic regions on the glycoside micelles and hydrophobic regions on the membrane proteins. The amount of glycoside in the complex varies depending on the glycoside used and the complex-bound membrane proteins and lies between 0.5 and 50% by weight, especially between 0.5 and 25% by weight, preferably between 0.5 and 15, often between 0.5 and 10, and especially between 2 and 8% by weight. If the charged antigenic monomeric proteins are separated from the solubilizing agent in the absence of the glycoside, protein micelles of the type produced according to EPC Application No. 81102213.6 are formed however.

It is suitable to remove the other fragments by gradient centrifuging since the sedimentation constant for the components involved decreases in the following order: cell fragment, protein complex with solubilizing agent or with glycoside, monomeric proteins and solubilizing agent. Thus, the other fragments can be removed with gradient centrifuging from the mixture of solubilizing agent, monomeric proteins, and other fragments before the glycoside is added and the solubilizing agent removed, e.g. by dialysis, gel filtration, chromatography or the monomeric proteins be separated from the solubilizing agent, e.g. by electrophoresis, chromatography or gradient centrifuging. In the latter method, it is also possible to remove the other fragments during the same gradient centrifuging, as the complex is formed. It is also possible to separate other cell components after the complex has been formed according to the above, e.g. by centrifuging, affinity chromatography or gel filtration.

The glycoside can be any glycoside at all with hydrophobic and hydrophilic regions. Preferably, the saponins are used which are described in R Tschesche and Wulf, Chemie und Biologie der Saponine in Fortschritte der Chemie Organischer Naturstoffe, published by W Herz, H Grisebach, G W Kirby, Vol 30 (1973), especially the strongly polar saponins, primarily the polar triterpensaponins such as the polar acidic bisdesmosides, e.g. saponin extract from Quillajabark Araloside A, Chikosetsusaponen IV, Calendula-Glycoside C, Chikusetsusaponin V, Achyranthes-Saponin B, Calendula-Glycoside A, Araloside B, Araloside C, Putranjia-Saponin III, Bersamasaponoside, Putranjia-Saponin IV, Trichoside A, Trichoside B, Saponaside A, Trichoside C, Gypsoside, Nutanoside, Dianthoside C, Saponaside D, preferably aescine from Aesculus hippocastanum (T Patt and W Winkler: Das therapeutisch wirksame Prinzip der Rosskastanie (Aesculus hippocastanum), Arzneimittelforschung 10(4), 273-275 (1960) or sapoalbin from Gypsophilla struthium (R Vochten, P Joos and R Ruyssen: Physico-chemical properties of sapoalbin and their relation to the foam stability, J Pharm Belg 42, 213-226 (1968), especially saponin extract from Quillaja saponaria Molina,

6

primarily the DQ-extract which is produced according to K Dalsgaard: Saponin Adjuvants, Bull Off Int Epiz 77 (7-8), 1289-1295 (1972) and Quil A which is produced according to K Dalsgaard: Saponin Adjuvants III, Archiv für die Gesamte Virusforschung 44, 243-254 (1974). Also mixtures of glycosides can be used. The amount of glycoside added should be at least 1-3 times their critical micelle formation concentration (CMC), preferably at least 5, especially at least 7-12 times. It is assumed that the glycoside in this case can be bound to and catch monomer forms of the membrane proteins. Preferably Quil A is used, which has a critical micelle formation concentration of 0.03% by weight. The amount of Quil A should then be at least 0.02% by weight, especially 0.05-0.5% by weight, preferably 0.2% by weight. The citations above concerning the glycosides are incorporated as references.

The separation of the charged monomeric antigenic proteins from the solubilizing agent has been done by centrifuging, dialysis electrophoresis, and different chromatographic methods.

### The centrifuge method

The mixture of fragmented cells or microorganisms and solubilizing agent made according to the above is gradient-centrifuged and layered on top of e.g. a sugar or salt solution, containing solubilizing agent, under which a gradient containing the glycoside is present, such as a sugar gradient or a gradient of glycerol or metrize amide or a heavy salt, e.g. CsCl (i.e. relatively inert substances which have suitable density, viscosity to act as gradient material), e.g. with the concentrations for a sugar gradient given below.

The gradient system is centrifuged at least at 100,000 g (depends on time and gradient, guided by practical circumstances). As sugar there can be used monosaccharides such as glucose, lactose, maltose, disaccharides such as saccharose, but also trioses, tetroses and glycerine. Preferably saccharose is used. The sugar concentration in the gradient is suitably at least 5, preferably 15-25% by weight as beginning concentration (uppermost in the gradient) and the final concentration is at least 20, preferably 45-60% by weight (lowermost in the gradient). The gradient can for example consist of an upper layer with 5-25% by weight sugar content and a lower layer with 20-60% by weight sugar content. However, there can also be several layers, the concentration differences between the individual layers being reduced correspondingly. The sugar gradient contains a glycoside or a mixture of glycosides. The amount of glycoside should be 1-3, especially at least 5, preferably at least 7-12 times CMC for Quil A, at least 0.02, especially at least 0.05-0.5, preferably at least 0.2% by weight. In this glycoside containing gradient, the solubility agent is separated, and the complex between the solubilizing agent and the protein is transformed to protein-glycoside complex.

On top of the sugar gradient there is a layer of a solution of sugar or heavy salt which contains solubilizing agent or a mixture of solubilizing agents; the lipids are remaining in thislayer. The concentration of solubilizing agent in this layer is less than or the same as in the applied mixture of microorganisms or cells and solubilizing agent and lies suitably between 0.25 and 3% by weight, preferably between 0.75 and 1.5% by weight, with 1% by weight being preferred. The sugar or salt concentration can be the same as or less than the concentration in the upper layer of the lower glycoside containing gradient, preferably 5-25% by weight, especially 15% by weight sugar.

After centrifuging at least at 100,000 g for at least 16 h, preferably for 20 h at 20° C, the proteinaceous fractions are collected and dialyzed against buffer (0.5 M to 0.001 M) preferably 0.005 M Tris-HCl, 0.01 M NaCl, pH 7.4 or 0.2 M ammonium acetate buffer, pH 7.0 and is concentrated e.g. as described in The Tools of Biochemistry by T G Cooper, edit John Wiley & Sons (New York 1974), e.g. by lyophilisation, vacuum dialysis and ultrafiltrating. During the centrifuging, all constituents are settled whereby the solubilizing agent loosens from the complex of protein and solubilizing agent, and the monomeric proteins are transferred to the glycoside and form complexes therewith. In the subsequent dialysis, the sugar is taken away.

The complex can possibly be purified further, e.g. from free glycoside by gradient centrifuging, e.g. by a sugar gradient containing 26-60% by weight sugar, preferably 10-40% by weight saccharose.

### The dialysis method

After purification of cells or the microorganisms as described above and after they have been mixed with solubilizing agent in the above described weight ratio, the mixture of cells and solubilizing agent, in the above described buffer can alternatively directly be mixed with at least 1-3, preferably 7-12 times CMC for Quil A 0.05-2% by weight glycoside, preferably 0.2% by weight glycoside, and dialyzed against the buffer such as 0.5-0.001 M, preferably 0.005 M Tris-HCl, 0.01 M NaCl, pH 7.4, especially 0.2 M ammonium acetate buffer, pH 7.0. This separates the solubilizing agent in the presence of the glycoside. The membrane protein complex produced can then be isolated with sedimentation gradient centrifuging, such as is described on page 14, second paragraph, the glycoside additive is excluded however, and is freed from the other fragments and free glycoside.

The mixture of cells and microorganisms and solubilizing agent in buffer can also be gradient centrifuged and e.g. be layered on a 5-60% by weight sugar gradient in the above buffer, preferably a 10-12% by weight saccharose gradient, and centrifuged at least at 150,000 g for at least 20 minutes, preferably for 30 minutes at 250,000 g. The other fragments are thereby separated from the complex between solubilizing agent and protein.

The proteinaceous upper liquid, called top fraction, is extracted and the glycoside is added in an amount of at least 1-3, preferably at least 7-12 times CMC for Quil A 0.05-0.5% by weight, preferably 0.2% by weight, and is dialyzed against buffer 0.5-0.001 M, especially 0.005 M Tris-HCl, 0.01 M HCl, pH 7.4, preferably 0.2 M ammonium acetate. The solubilizing agent is removed in the presence of the glycoside. Further purification can be done with sedimentation gradient centrifuging (see page 14, second paragraph). Further purification can be done by centrifuging through a sugar gradient containing 5-60% by weight sugar, preferably 20-50 or 10-40% by weight sugar.

## The electrophoresis method

Alternatively, the mixture of fragmented micro-organisms or cells and solubilizing agent or the proteinaceous top liquid (other fragments and free solubilizing agent removed) which is obtained, when this mixture is gradient-centrifuged e.g. by a 5-60% by weight, preferably 20-50% or 10-40% by weight sugar gradient in buffer, is separated by electrophoresis from the solubilizing agent and is transferred in a solution containing at least 1-3, preferably at least 7-12 times CMC, for Quil A 0.05-0.5% by weight glycosides, preferably 0.2% by weight glycoside. The charged monomeric antigenic proteins are thereby separated from the solubilizing agent. For separation by electrophoresis, it is suitable that the solubilizing agent-buffer solution not contain extra added salt which can interfere with the electrophoresis and produce excessively high temperature. It is possible to use e.g. zone electrophoresis with or without carriers and isotakophoresis with or without carriers. Common substances can be used as carriers such as polyacryl amide, agar, silica gel, starch, cellulose, polyvinyl chloride, ion exchanger, celite. Isolation and concentration of complexes are done as described on page 15, lines 26-28. Further purification with gradient-centrifuging (see page 15, lines 35-36, page 16 line 1).

If hydrophobic membrane proteins with various charges or weight are present in the starting material, it is possible with electrophoresis or the centrifuging methods to separate them from each other and produce separate complexes of them. With these conditions, it is possible to separate and enrich complexes of various membrane proteins.

## Chromatographic methods

The solubilized proteins can optionally, after being purified from cell fragments, be separated from the solubilizing agent with chromatographic methods, for example gel filtration, hydrophobic chromatography or affinity chromatography, e.g. ion exchange chromatography, the antigen structure being adsorbed into an insoluble substratum (matrix) which may consist of e.g. cellulose, agarose, dextrane, acrylamide and glass. Different ligands are coupled to the matrix structure which then receives specific properties which are utilized during the separation. The antigen structure is usually adsorbed at the same time as the solubilizing agent used passes unadsorbed through the matrix. Then follows desadsorbation of the antigen. Before or during the desadsorbation step there can take place an exchange of solubilizing agent, salt and buffer substance, the solubilizing agent being replaceable by the glycoside, and complex being formed.

In ion exchange chromatography, charged ligand molecules such as diethylaminoethyl (DEAE) are coupled to matrix and employed as cation exchangers. Carboxyl methyl (CM) or phosphate groups (P) are coupled to matrix and employed as anion exchangers. By using differences in net charge between antigen structures and solubilizing agent, these molecules are separated. In general, the solubilizing agent is uncharged and the protein charged. Elution is performed with salt gradient such as K- or NaCl- or pH adjustment with a suitable buffer, e.g. phosphate buffer in the presence of a solubilizing agent (as to concentration and examples see section Solubilizing above). In elution the protein can be purified, the solubilizing agent exchanged or the complex formed if the glycoside is added to the eluant instead of solubilizing agent. Salts are subsequently removed, e.g. by dialysis or gel filtration.

In gel filtration it is made use of the molecular weight of the solubilizing agent being smaller than the antigen structures and coming out in subsequent fractions. Upon complex formation the size of the antigen containing structures increases and they run away from the detergent containing zone.

By means of immunoaffinity-chromatography antibodies can be irreversibly bonded to the matrix mentioned above, whereafter the unique specificity and affinity of antibodies are utilized for purifying the desired antigen structure. The solubilizing agent has no affinity for antibodies. Elution is performed by mild denaturation, e.g. pH reduction to about 2.5 and in the presence of solubilizing agent or glycoside.

In lectin-chromatography are used lectins, a group of proteins capable of reacting reversibly with specific sugar groups, which makes it possible for them to bind glycoproteins, for example. The lectin is coupled as ligand to e.g. Sepharose (Pharmacia, Uppsala) or is commercially bought ready-coupled to a suitable matrix. Detergents (solubilizing agents) have no affinity for the immobilized lectin. The adsorbed antigen structure is usually desadsorbed by addition of low molecular sugar, possibly methylated, which has affinity for the lectin in question, such as mannose, methyl mannoside, glucose, methyl glycoside and N-acetyl glucosamine dissolved in buffered salt solution in the presence of solubilizing agent or glycoside.

In covalent chromatography, an antigen structure with a thiol group with a covalent bond is bonded to matrix. The thiol group in the antigen is selectively bonded to an activated thio group coupled to a suitable matrix by thio-disulfide exchange. This bond is reversible, and after removal by washing of the solubilizing agent, the thiol-carrying antigen structure can be eluted by reduction of the disulphide bond by mercapto ethanol or dithiotrietol in the presence of solubilizing agent or glycoside.

Hydrophobic chromatography

This technique employs the interaction of an immobilized hydrophobic ligand of the aliphatic or aromatic type such as alkyl, i.e. octyl or phenyl and hydrophobic surfaces of the protein or other antigen structure. One adsorbs at high ion strength by e.g. ammonium sulphate, and eluates at low ion strength with water or ethylene glycol.

When the complexes contain membrane proteins from bacteria, it being then advantageous to first break the cell walls before the cell material is treated by the process above. Examples of bacteria from which hydrophobic proteins can be produced are e.g. Escherichia, Staphylococci, Haemaophilus, e.g. H. influenzae, Bordetella, e.g. B. pertussis, Vibrio, e.g. V. cholerae, Salmonella, e.g. S. typhi, S. paratyphi, preferably adherence factor in Coli, e.g. pili K 88 and porin protein in e.g. Salmonella or outer membrane proteins from B. pertussis and Neisseria meningitidis.

Examples of usable viruses with envelopes are Orthomyxoviridae such as influenza A,B,C, Paramyxoviridae, especially measles virus, mumps virus, parainfluenza 1,2,3 and 4 viruses, canine distemper virus and rinderpest virus, Rhabdoviridae, especially rabies virus, Retroviridae, especially feline leukemia virus and bovine leukemia virus, Human T-cells Lymphotrophic virus HTLV 1,2 and 3, Herpesviridae, especially Pseudorabies, Herpes simplex I and II, Cytomegalo virus, Coronaviridae, Togaviridae, such as EEE, WEE, .VEE (Eastern, Western and Venezuela Equine Encephalitis), yellow feaver virus, especially bovine virus diarrhea virus, and European swine feaver virus Arenaviridae, Poxviridae, Bunyaviridae, especially Huntan virus, Iridioviridae, especially African swine feaver virus and among unclassified viruses, human hepatitis B-virus and Marburg-Ebola virus.

Examples of parasites which can be used according to the invention are Protoza, such as Toxoplasma, e.g. Toxoplasma gondii, Plasmodium, e.g. Plasmodium vivax, malariae, falciparium, Teileria parvum, ovale and Filaroidae, preferably Parafilaria and Onchocerca, Entamoeba histolytica, anaplasma of various types, Schistosoma such as Schistosoma haematobium, mansoni, japonicum, and Trypanosoma, e.g. Trypanosoma gambiense, brusei or congolesi.

b) It is also possible to start from hydrophobic non-membrane proteins or from non-hydrophobic proteins or peptides. Non-hydrophobic proteins or peptides can be rendered hydrophobic by coupling hydrophobic groups to them. The non-hydrophobic proteins may derive from viruses with or without envelope, bacteria, mycoplasma, parasites. Examples of non-enveloped viruses with non-hydrophobic proteins are Picornaviridae (also considered to have hydrophobic proteins) e.g. foot-and-mouth disease virus, polio virus, Adenoviridae, Parvoviridae, e.g. feline pest virus and swine parvovirus, Reoviridae, e.g. Rotavirus. Examples of mycoplasma are M. pneumoniae, mycoides, bovis, suis, hyorinos, orale, salivarium, hominis and fermentans.

These proteins or peptides can be obtained purified such as described under a) Purification and isolation.

The hydrophobic group that can be coupled to the non-hydrophobic proteins are straight, branched, saturated or unsaturated aliphatic chains, preferably having 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 carbon atoms, or hydrophobic amino acids or peptides or other hydrophobic structures such as steroids. The length of the hydrophobic structure is adapted to the size and nature of the protein. As an example, it can be mentioned that a peptide with 10-15 amino acids (foot-and-mouth disease virus) suitably is brought out with two tyrosine at the amino- or carboxy terminal end. A protein with a molecular weight of 70,000 daltons demands about 20 hydrophobic amino acids. Testing is made empirically. Thus, one uses especially peptides with 1 to 20 amino acids, preferably 1, 2, 3, 4, 5 amino acids, especially chosen among Trp, Ile, Phe, Pro, Tyr, Leu, Var, especially Tyr; cholesterol derivatives such as choline acid, ursodesoxycholine acid.

These hydrophobic groups must be bonded to a functional group that can be coupled to the non-hydrophobic protein. The functional group can be chosen from the ones mentioned on page 7, such as carboxyl-, amino-, disulphide-, hydroxyl-, sulfhydryl- and carbonyl group, such as aldehyde groups.

As coupling groups in hydrophobic structures are selected preferably carboxyl, aldehyde, amino, hydroxyl or sulfhydryl groups and peptides containing Cys, Asp, Glu, Lys. The hydrophobic groups with a group that can be coupled, must be dissolved in water with the aid of for example the solubilizing agents and detergents mentioned above or hydrochloric acid, acetic acid, caustic liquor, ammonia, depending on what substance is to be dissolved. pH is then adjusted to the neutral direction without the substance precipitating; here it is to make sure that there is not obtained a pH value that denaturates the protein to which the hydrophobic group is to be coupled.

The hydrophobic molecule is added to the non-hydrophobic protein in the molar ratio of 10:1 to 0.1:1, preferably 1:1.

Hydrophobic groups with a carboxyl group as coupling molecule can be coupled to the protein through water-soluble carbodiimides or mixed anhydrides. In the first case the carboxyl group is activated at pH 5 with carbodiimide and mixed with the protein dissolved in buffer pH 8 with a high phosphate content. In the latter case the carboxy compound is reacted with isobutylchloroformate in the presence of triethylamine in dioxane or acetonitrile, and the resulting anhydride is added to the protein at pH 8 to 9. It is also possible to convert the carboxyl group with hydrazine to hydrazide which together with aldehydes and ketones in periodate-oxidized sugar units in the protein gives hydrazone bonds.

The amino groups with nitrous acid can at a low temperature be converted to diazonium salts, which gives azo bonds with Tyr, His and Lys.

The hydroxyl groups with succinic anhydride can be converted to hemisuccinate derivatives which can be coupled as carboxyl groups.

Aldehyde groups can be reacted with amino groups in the protein to a Schiff's base.

The proteins or peptides so produced having received hydrophobic groups are then complex-bonded with glycoside, as described in a), but here the purification steps for removing cell fragments can be omitted.

c) It is also possible to start from hydrophilic proteins having enclosed hydrophobic groups and make them subsequently accessible by denaturating the proteins, i.e. with a low pH of about 2.5, 3M urea or at a high temperature above 70° C. Such proteins may be immunoglobulines such as IgG, IgM, IgA, IgD and IgE. The immunoglobulines can be used as antidiotypic antibodies. The proteins are obtained purified as proteins as described in b) and then complex-bonded to glycoside as described in a), the purification steps for removing cell fragments being omitted.

When starting from purified or synthetical proteins or peptides according to b) or c), they have a tendency to aggregate in the form of micelles during the preparation of iscoms. Therefore, the addition of one or more lipids, particularly cholesterol adds to the formation of the primary complex. The lipids are added to the protein or peptide as the solubilizing agents are added. The amount is not crucial. The molar ratio of lipid to protein or peptide should be at least 1:1. Then one of the four methods mentioned above can be used. When radioactive lipids are used, no radio-activity can be detected in the primary immunogenic complex.

Hydrophilic peptides/polypeptides can be covalently coupled to fatty acids incorporated into liposomes consisting of e.g. cholesterol, phosphatidylcholine and the fatty acids in a ratio of 1:7:2. The peptide/polypeptide is extracted from the liposomes with a detergent and separated from excess lipid by centrifugation on a sucrose gradient (10-30% sucrose) containing detergent.

Iscoms can be made as above, preferably with the centrifugation or dialysis method. By use of the centrifugation method, Triton X-100 can be used for solubilization of the liposome complex. By the use of the dialysis method, the detergent should be possible to dialyze away (e.g. Octylglycoside).

The immunogenic primary complex can be used for specific immuno-stimulation in humans and animals. They can thus be used as vaccines against deceases caused by bacteria, viruses, mycoplasmas and parasites and for producing antibodies for research purposes against membrane proteins from various animal cells.

Also mixtures of amphiphatic proteins from various bacteria or viruses can be added to produce vaccines against several afflictions. It can also be used as a carrier according to the invention.

II. Preparation of the complex according to the invention

The molecule against which antibodies are to be produced is coupled to the carrier molecule with the aid of lyophilized primary iscom or those solutions obtained when preparing the iscom by the centrifugation, dialysis, electrophoresis method, or with the aid of the afore-described chromatography methods, or those solutions remaining subsequent to further purification by centrifugation through saccharose solution.

When prepared in accordance with the centrifuge method, the primary iscom is obtained in a gradient-buffer mixture (see the centrifuge method), for example a sugar-buffer mixture. The sugar is removed by dialysis against or gel-filtration on, for example, Sephadex®G 50, to a buffer which is later used, when the primary iscom is coupled to the molecule to be made antigenic. Alternatively, there is used a volatile buffer, such as ammonium acetate, which departs when the mixture is subsequently freeze-dried.

When applying the dialysis method, the dialysis can be effected against the aforesaid buffers, or further dialysis, or gel-filtration can be performed to change the buffer.

When applying chromatographic or electrophoretic methods, the primary iscom is obtained in buffer solutions or, when the solutions are further purified, optionally in a gradient, for example in sugar-buffer solutions. These solutions can also be treated in accordance with the aforegoing, for buffer exchange or freeze-drying. The solubility of the molecule to be coupled to the primary iscom decides the pH at which coupling can be effected, and therewith also which coupling methods can be applied.

It is often known which groups in the molecule are relevant antigen determinants. The coupling reagent must not react with these determinants, but shall be selected so that other functional groups are reacted during the coupling process. Terminal amino groups or carboxyl groups are normally used in the case of natural peptides and proteins. Synthetic peptides can be provided with tyrosine groups or succinyl groups for example, and be coupled to primary amino groups via diazonium compounds or by adjusting the pH to approximately 8, respectively. The following coupling methods are preferred.

The functional groups comprise cystein groups coupled to one another by reaction with maleinimido-benzoyl-N-hydroxy-succinimide-ester, sulfohydryl groups, coupled to amino groups by diazotization, amino groups coupled to other amino groups with 4-hydroxy-3-nitro-methyl-benzimidate-hydrochloride, carboxyl groups coupled to amino groups via water-soluble carbodiimides or mixed anhydrides, amino groups, which have been converted to diazonium salts and bound via azo bonds to Tyr, His and Lys, hydroxyl groups which have been converted with succinic acid anhydride to hemisuccinate derivatives and coupled to carboxyl groups, aldehyde groups which have been reacted with amino groups to a Schiff's base, succinyl groups in synthesized peptides coupled to primary amines by pH-adjustment <5, hydroxyl groups which have been reacted with succinic acid anhydride to form hemisuccinate derivatives which have been coupled to carboxyl groups, vicinal OH-groups which have been converted to CHO-groups, for example with periodate and coupled to amino groups by adjusting the pH to >8, amino groups which have been coupled with glutaraldehyde to amino- or sulfhydryl groups with diimidoesters to amino groups, with diisocyanates to amino- or hydroxyl groups, amino-, hydroxyl- or sulfhydryl groups which have been coupled mutually two and two with methoxy-dichloride-triazine, amino- or hydroxyl groups which have been coupled to amino-, hydroxyl- or sulfhydryl groups with aryl-halogenides, sulfhydryl groups which have been coupled to the sulfhydryl or the amino groups with alkyl halogenides to amino groups with iodo-acetic acid and carbodiimide or to sulfhydryl groups with maleimides, aldehyde-, carboxyl- and amide groups which couple with hydrazine to primary amines under adjustment of the pH to 9-10.

An amino group can be coupled to another amino group by diazotization in the following manner.

The proteins or the peptides are each reacted per se with approximately 20mM of 4-hydroxy-3-nitro-methyl benzimidate-hydrochloride (MHNB) in approximately 0.1 M of a borate buffer (pH 9.0) at room temperature. The degree of protein modification with MHNB can be controlled by the reaction time. After 30 minutes the reaction solution is dialyzed against 0.1 M borate buffer overnight, at pH 8.0. The nitro groups are then reduced with approximately 1 mg/ml of sodium dithionite for from one to two minutes (1-2 mins) at room temperature. The reactants are removed on a Sephadex-G-25 column in an 0.1 M borate buffer, pH 8.0, or by dialysis against an 0.1 M borate buffer, pH 8.0, for 2-6 hours at +4° C, and against an 0.1 M borate buffer, pH 4, at +4° C, overnight. The amino groups are diazotized at 0° C with 0.1 M NaNO₂ at pH 4 for one (1) minute, whereafter the pH is quickly adjusted to 8.5. The other protein or peptide, preferably the iscom, is then added, preferably dissolved in a borate buffer of pH 8.5, at room temperature. Nitrite is removed by dialysis against an 0.1 M borate buffer, pH 8.0.

This method is preferably used when the molecule to be coupled to the primary iscom contains Tyr or His, which need not be coupled to MHNB. The molecule can be coupled directly to the MHNB-modified primary iscom and any unreacted molecule can be recovered. MHNB can be prepared in accordance with the Journal of Applied Biochemistry 1, 301-310 (1979).

11

Synthesized peptides can be provided with a terminal N-hydroxy succinimide peptide during the synthesizing process and coupled to primary amino groups in the iscom.

The succinylated peptide is dissolved at a low pH, pH <5, preferably in acetic acid. The primary iscom is then added, preferably dissolved in phosphate buffer, and the pH adjusted to approximately 8.0.

Tyrosine in one peptide can be coupled to tyrosine in another peptide by reaction with bis-diazobenzidine.

Sulfhydryl groups in peptides can be coupled to each other by reaction with maleinimido-benzoyl-N-hydroxy-succinimide esters.

The amino groups can be converted with nitrous acid at low temperature to diazonium salts, which produce azo-bonds with Tyr, His and Lys.

A carboxyl group in lipids, carbohydrates, peptides can be coupled to an amino group in peptides via water-soluble carbodiimides or mixed anhydrides. In the former case, the carboxyl group is activated at pH 5 with carbadiimide in a strong phosphate buffer approximately 10-15 mM and mixed with the primary iscom containing the protein, dissolved in a buffer, pH 8.0, having a high phosphate content (0.4 - 0.5, preferably 0.2 M). In the latter case the carboxy compound is reacted with isobutylchloroformate in the presence of triethylamine in dioxane or acetonitrile and the resultant anhydride is added to the iscom containing the amino group, at pH 8-9. The carboxyl group can also be converted with hydrazine to hydrazide, which in aldehydes and keytones in periodate oxidized sugar units present in the protein produce hydrazone bonds.

The hydroxyl groups, for example, in carbohydrates, can be converted with succinic acid anhydride to hemisuccinate derivatives, which are coupled to carboxyl groups in, for example, peptides. Vicinal OH-groups can be converted to a CHO-group with periodate for example, this CHO-group being subsequently reacted with a primary amine to a Schiff's base, by adjustment of the pH to above 8.0.

The molecule to be coupled is charged in a mole ratio to the protein in the primary iscom of 50:1 to 0.1:1, preferably 15:1 to 1:1. It may be suitable to optimate the immunogenicity of the conjugate for each coupled molecule primary iscom conjugate, by testing the same with different numbers of molecules coupled to each protein in the iscom. Depending upon the coupling method used, this can be controlled by varying the concentration of coupled molecules, the concentration of activating reagent (activates, converts the functional group), the activation period and the coupling time.

For reasons of a purely practical nature, the molecule to be coupled to the primary iscom is often charged in an amount corresponding to 1 mg per mg of protein, preferably 250-500 $\mu$g per mg protein in the primary iscom. The amount of protein in the primary iscom is determined, for example, by the Bradford-method (m.M. Bradford, Analyt Biochem 72 (1976)).

If when the linking is finished it is found by examination under electron microscope that the isomers have disintegrated into micelles, these micelles can be coupled together by a detergent, for example MEGA 9 and 10 (N-(D-gluco-2,3,4,5,6-pentahydroxyhexyl)-N-methyl-non-amide and -methyldecanamide, respectively, prepared in accordance with Biochem J (1982) 207, 363-366) or $\beta$-octylglucoside or some other detergent, for example those recited on pages 9 and 10, in mixture with glycoside, for example Quil A. Surplus reagent and any detergent and glycoside present are then removed by dialysis against, or gel-filtration through a suitable buffer, for example PBS, or a volatile buffer, such as an ammonium acetate buffer, which departs when the final product is freeze-dried, this freeze-drying of the product being an optional step.

The novel antigenic complex can be stored in a freeze-dried state or in aqueous-suspension form. The invention also relates to human and veterinary medical compositions containing as an active substance one or more complexes according to the invention, optionally together with conventional additives and extenders, preferably in the form of a buffer solution of iscom in, for example, a TN-solution (cf Example 1) or in a physiological salt solution, for example 0.1 M NaCl, pH 7.2 - 7.6. The pH can be adjusted with 0.05 M Tris-HCl.

The complexes were used for immunizing human beings and animals in an amount corresponding to approximately 0.1 $\mu$g - 1 mg complex per individual on one or two occasions (with a two week interval therebetween).

The invention will now be described in more detail with reference to a number of Examples.

Example 1. Parainfluenza-3-virus U23 isolated in Umeå, was purified by means of centrifuging through 30% by weight saccharose at 100,000 g for 1 hour at 4° C. The bottom layer was dissolved to a concentration of about 10 mg/ml in 0.05 M Tris-HCl, pH 7.4 and 0.1 M NaCl (TN). 1-5 mg/ml PI-3-virus in the same buffer (TN) was solubilized with 2% by weight (final concentration) Triton X-100 together with about $10^5$ counts/minute $^3$H-marked virus (A Luukkonen, C Gamberg, E Renkonen (1979) Virology 76 pp 55-59, which is incorporated as a reference) in TN buffer. A sample volume of about 200 $\mu$l was layered on 300 $\mu$l 15%

saccharose containing 1% Triton X-100 in TN and a 12 ml saccharose gradient in TN from 20 to 50% by weight containing 0.2% by weight Quil A. The centrifuging was carried out at 250,000 g for 22 hours at 20°C. After centrifuging, fractions of 500 $\mu$l were collected from below and samples (20-50 $\mu$l) were measured for radioactivity. The radioactive protein fractions were put together and dialyzed on 0.005 m Tris-HCl, 0.01 M NaCl, pH 7.4, was dosed in 10 ml flasks and concentrated by lyophilisation for 18 hours in an Edwards freeze-drying apparatus.

This preparation had a sedimentation coefficient of 24 S.

Further purification of the complex was done by centrifuging of the complex through a 10-40% by weight saccharose gradient.

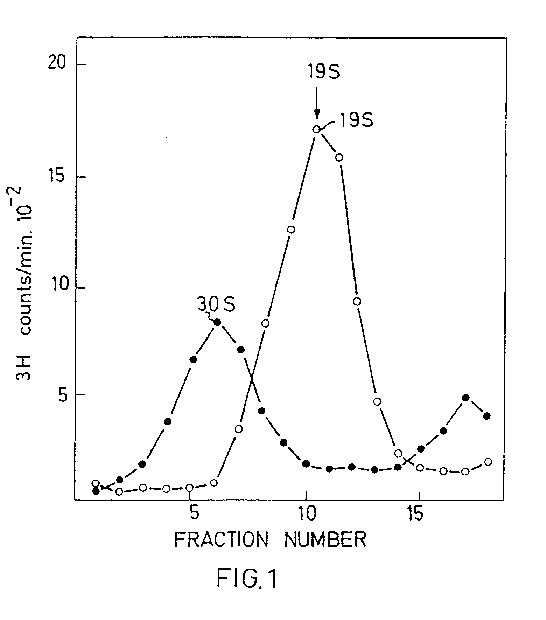

Example 2. The process according to Example 1 was repeated by using equine influenza virus (Solvalla, isolated from Solvalla, Stockholm). The experiment was repeated without glycoside being added (i.e. in principle according to EPC application 81102213.6) and the protein micelles so obtained and the protein complex produced with glycoside were subjected to sedimentation gradient centrifuging through a 10-40% by weight sugar solution at 280,000 g for 4 hours at +4°C. The results are given in Fig 1 which also shows the sedimentation coefficient for tyroglobulin as standard (19 S at the arrow). It reveals that the sedimentation coefficient for protein micelles is 30 S [●] and for glycoside protein complex 19 S [o]. (The virus glucoprotein was marked with galactosoxidase-³H-borhydride method.)

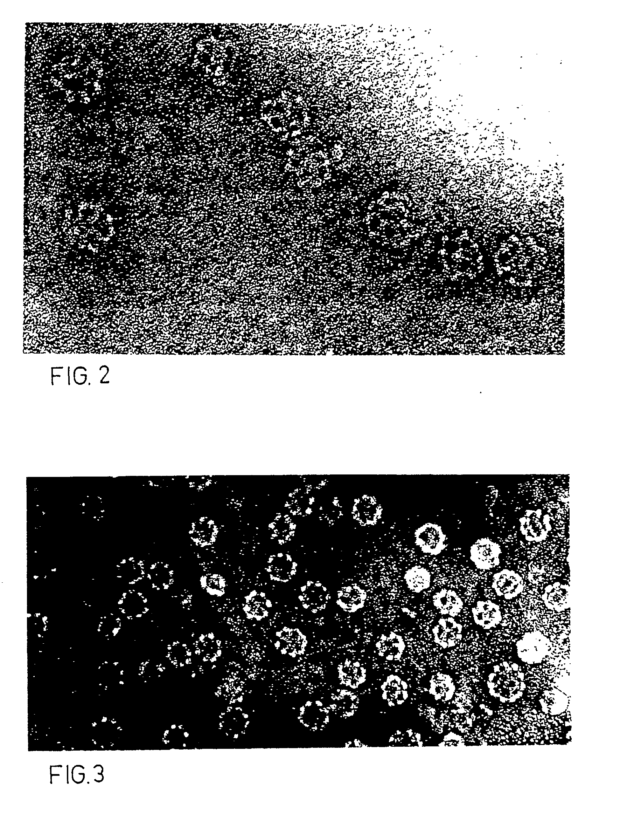

Example 3. The process according to Example 1 was repeated with measles virus instead of parainfluenza-3-virus. A complex was obtained which under electron microscopy showed the characteristic structure revealed in Fig 2.

Example 4. Rabies virus produced at Riv Bilthoven (Holland) according to the method described by Van Wezel et al, Develop Biol Standard (1978), 41, 159-168, were concentrated to 2 mg/ml in TN. 1 mg of virus was made soluble with 100 mM octyl-$\beta$-D-glucoside and was incubated for 45 minutes at 20°C. The sample was layered over a 50% by weight sugar solution and was centrifuged at 250,000 g for 45 minutes at +4°C. The upper solution was extracted and Quil A was added to 0.2% by weight.

The sample was enclosed in a cellulose hose and dialyzed at +20°C in 0.15 M ammonium acetate buffer in a volume of 1000 ml, which was changed 3 times during 72 hours of constant agitation. The dialyzed material contains the rabies virus complex. A portion of the material was purified further by means of centrifuging through a 10-40% by weight sugar solution at 280,000 g for 4 hours at +4°C. Electron microscopy revealed the structure shown in Fig 3.

Example 5. The process according to Example 4 was repeated with measles virus. The complex obtained showed the same structure as the complex produced according to Example 3.

Example 6. Parainfluenza-3-virus (U-23) was purified with saccharose gradient centrifuging and virus thus purified was dissolved to a concentration of 10 mg/ml in 0.02 M barbiton buffer, pH 8.0, 0.24 M glucose (BG). 1-5 mg/ml PI-3-virus in BG-buffer was made soluble with 2% Triton X-100 together with about 10⁵ ³H-counts/minute-marked virus (according to ref Luukkonen et al, 1977) in BG-buffer. A sample volume of 1 ml was layered on a 1% agarose gel containing 0.02 M barbiton buffer, pH 8.0, and 0.02% by weight Quil A. The agarose gel was enclosed in a tube with a surface of 85 mm² and a height of 25 mm. The upper portion and the lower portion of the tube were each connected to electrophoresis buffer of 0.02 M barbiton buffer, pH 8.0. The upper vessel was connected to a negative electrode and the under vessel to a positive electrode. The electrophoresis was carried out at 5 V/cm for 4 hours. The sample was collected on the anode side of the gel and it was measured for radioactivity. The sample was dialyzed in 0.15 M ammonium acetate buffer, pH 7.0, and was concentrated by lyophilization.

This preparation had a sedimentation coefficient of about 20 S, measured in the same manner as in Example 2.

Further purification of the complex was done by centrifuging the complex through a 10-40% by weight saccharose gradient.

Example 7. Stomach fluid obtained from mice infected with Toxoplasma gondii was filtered through gauze and cotton in a 10 ml syringe, centrifuged at 1000 rpm for 20 minutes, and the cell-pellet was dissolved in PBS and washed twice (1000 rpm 20 minutes). The end pellet, approximately 1-10 mg protein, was extracted (three times one hour r.t.) with 1 ml 5% MEGA.

The three overlying liquids were combined and centrifuged for 10 minutes at 1000 rpm, and 0.1% Quil A was added to the overlying liquid. The mixture was then dialyzed against a 0.05% ammonium acetate for 48 hours.

Example 8. Bacteria E. coli with plasmid pili K 88 were shaken mechanically and precipitated three times at the isoelectric point. The material was then treated in the same manner as described in Example 1. Complexes were obtained with the characteristic structure shown in Figs 2 and 3.

Example 9. The process according to Example 8 was repeated with Salmonella, which carries porin protein.

Complexes were obtained with the characteristic structure shown in Figs 2 and 3.

Example 10. Epitel kidney cells from cats which had been infected by Feline leukemia virus were treated with the process according to Example 1. Complexes were obtained with the characteristic structure revealed in Figs 2 and 3.

Example 11. Epitel kidney cells which had been transformed by bovine leukemia virus were treated with the process according to Example 1. Complexes were obtained with the characteristic structure shown in Figs 2 and 3.

Example 12. Parainfluenza-3-virus U-23 was purified and protein complex was prepared according to the process of Example 1 but with the difference that saccharose gradient in TN from 20 to 50% by weight contains a saponin other than Quil A. Two commercially available saponins were tested, Merck "Weiss", rein 51400023 and Sc, Lickhardt "S" Schuchardt, Munich. (Saponins in pure form. The manufacturer did not want to reveal the type of the product. In thin layer chromatography they differ from Quil A). The resulting complex had a sedimentation coefficient of 24 S and showed the same structure as that of the complex prepared according to Example 3.

Example 13. 5 mg measles virus were solubilized according to Example 3 and applied to an anion exchanger of DEAE cellulose type. The ion exchanger was kept in a column of 20 ml and was equilibrated with 0.01 M phosphate buffer, pH 7.2, 0.5% by weight of octyl-$\beta$-D-glucoside. The sample material was applied to the ion exchanger and non-adsorbed material was washed away after rinsing-through by 5 column volumes 0.01 M phosphate buffer, pH 7.2, 0.5% by weight of octyl-$\beta$-D-glucoside, and then the adsorbed material was eluted after addition to the column of a salt gradient between 0 and 0.5 M NaCl dissolved in 0.01 M phosphate, pH 7.2, 0.5% by weight of octyl-$\beta$-D-glucoside. The fractions in which measles membrane proteins were identified, were combined and Quil A was added to 0.1% by weight and dialyzed on 0.05 M ammonium acetate, pH 7.0. A complex was formed with the characteristic structure shown in Figs 2 and 3.

Example 13a. A measles virus (RIV-Bilthoven) was solubilized in accordance with Example 3. The virus-detergent was centrifuged for two hours at 100.000 x g. The resultant upper liquid was dialyzed against a phosphate buffer of low salt content (10 mM phosphate - 50 mM NaCl-pH 7.2) and applied to a DEAE-Sepharose-anion exchanger, which was in equilibrium in 10 mM phosphate - pH 7.2 - 50 mM NaCl - 0.05% TX-100. Non-absorbed material was washed away with the same buffer. The column was then brought into equilibrium with a buffer containing 10 mM phosphate - 50 mM NaCl - 0.1% Quil A. Absorbed material was eluted with the same buffer containing 300 mM NaCl. The eluated material contained complexes having the characteristic structure of Fig 2.

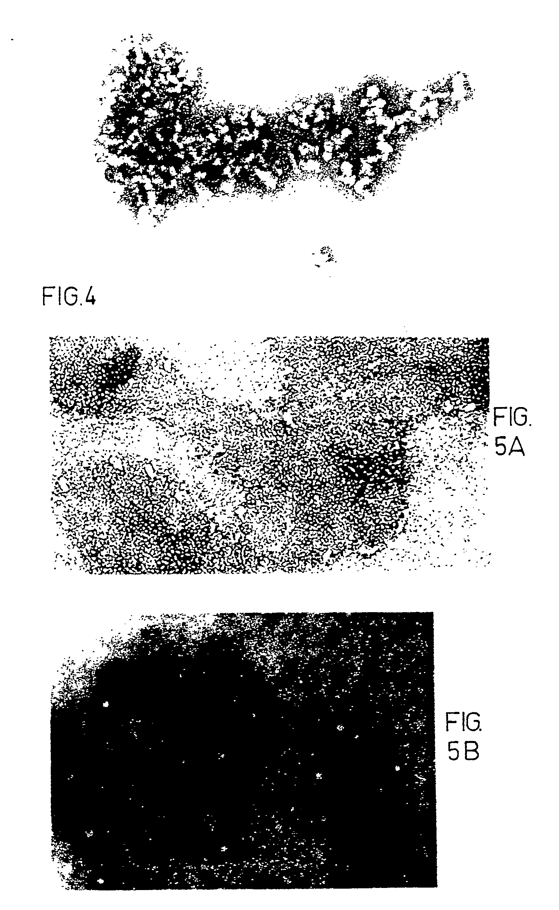

Example 14. 22 nM particles of Hepatitis B virus received from London School of Tropical Medicine and Hygiene (England) was resuspended to a concentration of 1 mg/ml in TN. 0.3 mg protein of 22 nM particles were solubilized by 2% by volume of Triton X-100, 0.5 M NaCl and incubated for 16 hours at $+37^\circ$ C. Then the process according to Example 1 was repeated. The resulting complex showed a sedimentation coefficient of 20 S. Electron microscopy revealed a complex having a structure as that shown in Fig 4. This structure differs from the structure shown in Fig 2 in that it consists of parts of this structure.

Example 15. 3 mg bovine diarrhoea virus (BDV) dissolved in TN, were solubilized by addition of Triton X-100 to 1% by volume. The mixture was agitated for 2 hours at room temperature. Thus solubilized virus was applied to a lectin column consisting of the lectin Lentil immobilized to Sepharose 4 B (Pharmacia, Uppsala). The column was equilibrated with TN and after introduction of the virus material on to the column, it was washed with 5 column volumes TN containing 0.1% by volume Triton X-100 followed by 10 column volumes TN. Virus enveloped proteins were desadsorbed by eluating buffer consisting of 0.2 M methyl-$\alpha$-D-mannoside, 0.5% by weight of octyl-$\beta$-D-glucoside dissolved in TN being added to columns. The fractions containing virus enveloped proteins were collected and Quil A was added to 0.1% by weight. The mixture was dialyzed on 0.05 M ammonium acetate pH 7.0 at $+4^\circ$ C for 3 days with three changes of buffer volume of 1 litre.

The final product was subjected to lyophilization and electron microscopy revealed (complex) structures being parts of the complex shown in Fig 4. This preparation had a sedimentation coefficient of 20 S.

Example 16. Purified, killed poliovirus prepared at RIV-Bilthoven in accordance with the method described by Van Wezel et al (Develop Biol Standard (1978) 41, 159-168), was solubilized in a suitable buffer, such as TN, containing a solubilizing agent, for example 2% sodium dodecyl sulphate, by incubation at $37^\circ$ C for two hours. The virus capcide proteins were separated by electrophoresis in a suitable 10% polyacrylamide gel containing 0.1% sodium dodecyl sulphate. Subsequent to identifying the positions of the proteins in the gel, suitable strips were cut therefrom, and the proteins eluted by electrophoresis. VP-3 having a molecular weight of approximately 26 K Dalton is one of the capcide proteins. Triton X-100 was admixed with a VP-3-containing solution, to a final concentration of 2%. This mixture was then used in the preparation of protein complexes (iscom) in accordance with the centrifugation method of Example 1.

Electron microscopy revealed the characteristic structure of the preparation illustrated in Fig 3.

Example 17. Purified polio virus which was killed with formalin (produced at RIV Bilthoven) was dissolved in 67% by volume of acetic acid containing 0.1 M $MgCl_2$. The virus material was then subjected to ultra-centrifugation for 1 hour at 100,000 g, and the supernatant containing solely virus proteins was taken care of and dialyzed in the presence of 0.1% by weight Quil A against 0.01 M Tris, 0.14 M NaCl, pH 7.4. The resulting complex showed the same structure as that of the complex prepared according to Example 3.

Example 18. Outer membrane proteins of Neisseria meningitidis were received freeze-dehydrated from National Institute of Health (the Netherlands) and were dissolved in TN containing 2% by weight octyl-$\beta$-D-glucoside and 0.1% by weight Quil A, and treated in the same manner as in Example 4. The resulting complex had a sedimentation coefficient of 20 S measured in the same manner as in Example 2.

Example 19. Peptide with hydrophobic amino acids. Foot-and-mouth disease peptide 144-169, O Kaufbehren. VP 1 synthesized with 0, 1, 2, 3 and 4 Tyrosin, coupled to one end, was used (as commercially received). The peptide was dissolved in an exceedingly small amount of 67% by volume of acetic acid, neutralized with 25% ammonia and diluted to 0.5 M Amm Acetate with Ag dest (detergent to 2% final concentration). 0.1% glycoside was added and the mixture was dialyzed on 0.05 M Amm Acetate, pH 7. (Dialysis tube Spectra Por 6 MWCO 1.000).

Complex having formed can be shown by electron microscopy (see Fig 5) revealing a spherical electron tight particle having a length 20-40 nm and a breadth 10 nm. Other sizes were also seen.

Fig 5a shows the electron microscope (EM) picture of the peptide coupled to three tyrosine and Fig 5b shows the EM picture of the peptide coupled to four tyrosine.

Example 20. IgG from mice, purified according to known methods (M Van den Branden, J L de Coen, L Kanarek and Royschaert (1981) and Molecular Immunology 18, pp 621-631 (1981)) or enriched by ammonium sulphate precipitation (J E Conradie, M Govender, L Visser, Journal of Immunological Methods, Vol 59, pp 289-299 (1983); citations incorporated as reference) was dialyzed overnight against 1 litre 0.15 M phosphate citrate (PC) buffer, pH 2.5, in refrigerating chamber. 2% detergent (e.g. octyl-$\beta$-D-glucoside) was then added. If a detergent having a low critical micellar concentration (MCM) is used, detergent should be changed before starting dialysis. The mixture was dialysed on PC pH 7. One hour later, Quil A was added to final concentration of 0.05%, and the dialysis was continued on PC pH 7 for 24 hours in refrigerating chamber.

Complex forming was shown by centrifuging through a 5 to 30% saccharose gradient for 3.3 hours at 40,000 rpm in a Beckman SW-60 rotor. The complexes were detected in the gradient by means of ELISA technique.

Example 21. Preparation of iscoms from Avian Bronchitis-virus (family Coronaviridae). Five mg of sucrose gradient purified avian bronchitisvirus in 0.5 ml TN-buffer (0.05 M Tris and 0.1 M NaCl) were solubilized by adding the detergent octyloglucoside to 2% and incubation for 1 h at 37° C and 2 h at room temperature. The mixture was layered on top of a sucrose gradient consisting of a 3 ml 30% sucrose in TN in the bottom of the tube and above that 1.5 ml of 10% sucrose in TN containing 1% octyloglucoside and 0.1% Quil A. The centrifugation was performed in a TST 54 Contron rotor for 2 h at 50,000 rpm and at 20° C.

The two ml on the top of the gradient were collected and dialyzed at a temperature of 6° C against 1 liter 0.05 M ammonium acetate, which was replaced three times during a period of three days. After the dialysis the preparation was centrifuged through a 10 ml 10% sucrose in TN for 6 h at 40,000 rpm in a TST 41 Contron rotor at 20° C. The pellet was dissolved in 1 ml TN buffer.

Electron microscopy showed the typical morphology of iscoms (see Fig 2).

Example 22. Preparation of iscoms from Feline Leukemia virus (family Retroviridae). For the production of FeLV either the FL74 or the F422 lymphobastoid cell line was cultured in suspension according to Wolff et al. The medium was collected at daily intervals and cell debris was removed by low speed centrifugation. It was concentrated by ultrafiltration using a Millipore[R] filter system (exclusion MW 100,000) and the virus was further purified by ultracentrifugation twice through a 30% (w/w) sucrose layer. The resulting pellet was solubilized with TN buffer (0.05 M Tris and 0.1 M NaCl, pH 7.4) containing 2% Triton X-100 and 0.02% dithiobis-n-nitropyridine. The iscoms were prepared with a centrifugation method as previously described. Briefly 200 $\mu$l of the solubilized virus was applied onto a layer of 200 $\mu$l 8% sucrose in TN containing 1% Triton X-100 which was layered over a linear 5 ml 10-40% sucrose gradient containing 0.2% Quil A. The centrifugation was performed in a SW50 rotor at 150,000 g for 4 hrs at 20° C. The gradient was collected in 500 $\mu$l fractions and the gp 70/85 containing fractions were identified by 10% SDS polyacrylamide gel electrophoresis (SDS-PAGE). The presence of gp 70/85 was further confirmed in a dot blot system. Samples of 5 $\mu$l of the fractions were applied to a nitrocellulose filter, soaked for 30 minutes in TN buffer containing 5% albumin and washed with TN buffer. A horse radish peroxidase (HRP) labelled monoclonal

EP 0 180 564 B1

anti gp 70/85 antibody was then applied and binding was demonstrated after washing in TN buffer. The presence of iscoms was confirmed by negative contrast electron microscopy as previously described . Total protein content was measured according to Lowry , and the amount of gp 70/85 was estimated by scanning of the polyacrylamide gel. Preparations were lyophilized for storage.

In immunization experiments in cats the iscoms induce neutralizing antibodies and protection against infection of a challenge experiment .

Example 23. Preparation of iscoms from Plasmodium Falciparum (Protozoa). One mg plasmodia falciparum (trophozoites, schizonts and merozoites) was suspended in 500 $\mu$l TN buffer containing 1% MEGA. Cells and cell rests were spun down by low speed centrifugation at 3,000 rpm for 30 minutes at room temperature. The suspension was incubated for 1 hr at 30°C and 2 hrs at room temperature. After that the suspension was layered on top of a dicontinuous sucrose gradient consisting of 3 ml of 30% sucrose in TN buffer in the bottom of the tube. Above that was 1.5 ml of 10% sucrose and 1% MEGA in TN buffer and 0.1% Quil A. The gradient was centrifuged for 3 hrs at 50,000 rpm at 20°C in TST 55 Contron rotor. Three ml on the top of the gradient were collected and dialyzed against one liter 0.1 M ammonium acetate which was replaced 3 times during the 3 days of dialysis. After that the preparation was collected and layered above 10% sucrose in 13 ml centrifuge tube and centrifuged for six hours at 20°C at 40,000 rpm in a TST 41 Contron rotor. The pellet was dissolved in 500 $\mu$l TN buffer. Electron microscopy reveal particle with the typical morphology of iscoms.

Example 24. Preparation of iscoms from outer membrane proteins of Bordetella Pertussis. 10 g of lyophilized Bordetella pertussis was suspended in 340 ml distilled water. The bacteria were disintegrated in a French press. After that the suspension was heated to 66°C and an equal volume of phenol water solution (90% phenol and 10% distilled water) with a temperature of 66°C was added. The mixture was put on a shaker for 20 minutes and then cooled to 5°C and centrifuged for 20 minutes at 3000 rpm. The water phase was collected and MEGA was added to a concentration of 2% and SDS to a concentration of 0.1%. Thereafter the preparation was centrifuged for 20 minutes at 3000 rpm and the supernatant collected. The mixture was concentrated by ultrafiltration using a Millipore[R] filter system (exclusion MW 100,000) and the protein concentration measured by Lowry (1) was adjusted to 20 mg protein per ml. Thereafter, Quil A was added to a concentration of 0.2% and the suspension was dialyzed at 6°C against one liter 0.15 M ammonium acetate replaced three times during a period of three days. Electron microscopy revealed particles with the typical morphology of iscoms.

Example 25. 10 mg of HINI-PR8 virus was solubilized with 100 $\mu$l 20% N-Decanyl-N-methyl-glucamin and incubated for 1 hour at room temperature. The solubilized membrane proteins were separated from the core structure, by centrifugation through 20% sucrose containing the detergent at a concentration above the critical micellar concentration. The membrane proteins were collected and Quil A added to a final concentration of 0.1% and dialyzed extensively against 0.9% NaCl the first 4-6 hours at room temperature, then at +4°C.

Example 26. 300 $\mu$g of gp 340, an envelop protein from Epstein Barr virus (a herpes virus) in 200 $\mu$l PBS, is added to tube where 70 $\mu$g of cholesterol is dried to the wall. Triton X-100 is added and the mixture is kept at room temperature for 2 hrs. The mixture in a volume of 300 $\mu$l is layered on top of a gradient, which from the top to the bottom consists of 200 $\mu$l 15% sucrose with 1% TX-100 and ~ 12 ml of 20% sucrose in PBS containing 0.1% OA. The centrifugation was done at 40,000 rpm in a Beckman SW 40 rotor for 16 hrs and 20°C. The gradient was collected from the bottom in 500 $\mu$l portions. The gp 340 mixed with detergent and cholesterol can be reconstituted into iscoms by the dialysis method. In that case Quil A was added to a final concentration of 0.1% before dialysis. It is then preferable to have a detergent which easily can be dialyzed away, e.g. octylglycoside which was used in this case.

The mixture was then dialyzed for 48 hrs at 4-6°C against PBS (also TN buffer has been used). The formation of iscoms was verified in electron microscopy. The advantage of using e.g. envelope proteins of different virus purified with different methods, i.e. the method of choice is obvious.

Example 27. Iscoms made from hydrophobic peptides. Iscoms made from peptides with a hydrophobic tail e.g.

Tyr$_3$-FMD

Tyr$_4$-FMD

palmitic acid-FMD

myristic acid-FMD

Dissolve 1 mg of peptide in 100 $\mu$l 20% octylglucoside, N-Decanyl-N-methyl glucamine or any of the dialysable detergents,

add an equimolar (±20%) amount of cholesterol in 50 $\mu$l detergent-buffer solution,

add buffer and Quil A to make a final concentration of 1 mg/ml peptide and 0.1% Quil A,

16

dialyze (dialysis bag cut of $M_w$ 1000) extensively against PBS or any other suitable buffer, the first 24 hrs at room temperature.

Example 28. A sequence of amino acids 139-153 representing a part of a growth factor for T-cells Interleukin II was synthesized with two tyrosines in the amino terminal.

The peptide was coupled to glycol proteins in iscom, by diazotization.

The iscom comprised glycoproteins derived from influenza virus, strain H2 N2/PR-8, and was prepared by centrifugation in accordance with the method disclosed in Examples 1 and 2 above.

1 mg of iscom preparation (calculated as the amount of protein in the iscom) was dissolved in a 0.1 M borate buffer, pH 9.5 (1 mg/ml). Activation was effected by adding 5 mg (5 mg/ml.mg) of solid MHNB (methyl-4-hydroxy-3-nitrobenz-imidate). The mixture was shaken until all the MHNB had dissolved, whereafter the solution was incubated for two hours at room temperature (Ref Müller, Pleiderer, Hoppe-Seyler's Z Physiol Chem, Vol 359, pp 407-411 (1978), Muller, Pleiderer, Journal of applied Biochem, Vol 1, pp 301-310 (1979)), The mixture was then dialyzed overnight against 0.1 M borate, pH 8.0, at +4° C, reduced with dithionite (1 mg dithionite/ml) for 1-2 minutes at room temperatures, dialyzed against 0.1 M borate, pH 8.0, for 2-6 hrs at +4° C, and dialyzed against 0.1 M borate, pH 4.0, overnight at +4° C.

The mixture was then diazotized by adding $NaNO_2$ (6.9 mg/ml) from a strain solution (100 mg/ml in Aq dest), incubated for one (1) minute on an ice bath, whereafter the pH was adjusted rapidly to 8.5 (borax and NaOH). 1 mg peptide dissolved in 50-100 $\mu$l 0.1 M borate, pH 8.5, was then added to the solution and thoroughly mixed therewith, whereafter the temperature was raised to room temperature and the solution then incubated for two hours.

The mixture was then dialyzed against 0.1 M borate, pH 8, overnight at +4° C. Dialysis hose cut-off = 1000. 1% MEGA (or 2% $\beta$-octylglucoside) + 0.1% Quil A were then added, whereafter the mixture was incubated for 30 minutes at room temperature. The mixture was then dialyzed through PBS (conventional dialysis hose) for 2-4 hours at room temperature and then at a temperature of +4° C overnight. Electron-microscopy confirmed that iscom complexes were present.

Five mice were each immunized with approximately 10 $\mu$g of the peptide in the form of the iscom complex prepared in accordance with a method described by Müller, J, Pfleiderer, G A (1979), Journal of Applied Biochem I, pp 301-310. Subsequent to immunizing the mice, the antibody response in serum was evaluated by the ELISA-technique, where the enzyme alkaline phosphatase was used. The background, i.e. the readability, at absorbance 405 was 0.01% or lower with serum samples deriving from mice which had not been immunized. Serum taken from the immunized mice contained antibodies which could be shown in dilutions up to 1:100, or still higher, and with reading values which were ten times greater than the background, or even greater. No antibody response was observed, when immunizing solely with peptides.

Example 29. A sequence of 20 amino acids (141-160 obtained from VIP FMD virus (foot-and-mouth disease) was synthesized in three variants:

1. 15 amino acids without further additives;
2. 15 amino acids plus two (2) additional tyrosines in the amino terminal;
3. 15 amino acids plus one (1) N-hydroxy succinimide ester.

The peptides were coupled to iscom produced according to Example 1 from glucoproteins from influenza virus strain H2N2/PR-8 with three coupling methods.

Variant 1 were coupled to iscoms via their COOH-terminals, using the carbodiimide method (cf carbodiimide coupling).

250 $\mu$g peptide were dissolved in 250 $\mu$l of a 25 mM phosphate buffer, pH 5.

2.5 mg EDC (1-ethyl-3-(3-dimethylaminopropyl)carbodiimide sigma) were dissolved in 250 $\mu$l Aq Dest and mixed with the peptide. The mixture was then incubated for two minutes at room temperature.

1 mg iscom (calculated as protein) dissolved in 500 $\mu$l of a 0.4 M phosphate buffer, pH 8, was then added to the mixture and reacted overnight at room temperature. The mixture was then dialyzed overnight against PBS at +4° C.

The coupling yield was 27%, which corresponds to approximately five (5) FMDV-peptides for each protein in the primary iscom.

Variant 2 were coupled to iscoms via their tyrosines through diazotization, cf sample 28. 55% of the peptide was coupled to the primary iscom, which corresponds to approximately 13 peptides per protein molecule in the primary iscom.

Variant 3 coupling of N-hydroxy succinimide-peptide to iscoms. 1 mg of iscom preparation (H2N2/Pr-8) of influenza virus produced by the centrifugation method (cf diazo-coupling) was dialyzed against 0.05 M phosphate buffer, pH 5.

1 mg succinyl-peptide was dissolved in 25 $\mu$l of concentrated acetic acid, whereafter the peptide + iscom-preparation was mixed and titrated for 2-3 minutes with minor portions of 0.2 M $Na_2HPO_4$

to pH 8. The mixture was then incubated for 4 hrs at room temperature.

1% MEGA 10 (or 2% β-octylglucoside) and 0.1% Quil A were then added and the mixture incubated for 30-60 minutes at room temperature, whereafter the mixture was dialyzed against PBS for 2-4 hours at room temperature and then overnight at +4° C.

Example 30. Biotin-iscom complex. The iscom was formed from glucoproteins obtained from influenza virus serotype A, subtype AEq2 BEq2 strain Solvalla/79. The iscom was prepared in accordance with Examples 1 and 2.

1 mg (calculated as protein) of Solvalla iscom was dissolved in 1 ml 0.1 M NaHCO₃, pH 8, and added with 100 µl (0.2 mg from strain solution 2 mg/ml in DMSO) N-hydroxy succinimido biotin (Sigma), whereafter the resultant solution was incubated for 4 hours at room temperature and dialyzed overnight against PBS at +4° C. Electron-microscopy revealed the presence of iscom complexes.

Three mice (Balb/c) were each immunized twice with 50 µg of the conjugate (calculated as the total weight of the conjugate) at 14-day intervals. Three days after the first immunization, all mice were found to have an antibody titer of ≧ 1/100,000 measured according to the ELISA technique. The wells in the ELISA plates were coated with biotinylized BSA (bovine serum albumin). BSA was used to expose the biotin during the analysis. None of the antibodies taken from the immunized mice reacted with plates coated solely with BSA. The animals were not seen to suffer any side effects.

Example 31. Coupling of peroxidase to PR8-primary iscom according to Nakane Kawaoi (1974) Peroxidase-labelled antibody. A new method of conjugation, I Histochem Cytochem 22 1084.

1 mg HRP (peroxidase) was dissolved in 200 µl 0.3 M NaHCO₃, pH 8.1, and 20 µl 1% FDNB (fluorodinitrobenzene) were added and incubated for 1 hour at room temperature, for blocking of the primary amino groups. 200 µl 0.08 M NaIO₄ were then added and incubated for 30 minutes at room temperature, to convert vicinal OH-groups to CHO-groups. 200 µl of 0.18 M ethylene glycol were added and incubated for one (1) hour at room temperature to remove unreacted NaIO₄.

The activated peroxidase and 1 mg (calculated as protein) influenza virus strain H2N2 PR-8 iscoms (prepared in accordance with Example 1) were then dialyzed overnight against 0.01 M NaHCO₃, pH 9.5 (each per se).

The PR-8 + HRP-solutions were mixed and incubated for at least four hours at room temperature. The conjugate was cleansed from non-coupled HRP by gradient centrifugation on saccharose 10-40%, the HRP-activity following the iscoms. Electron-microscopy revealed the presence of iscom complexes.

Coupling of the molecules in the above Examples was controlled by gradient centrifugation. Radioactively labelled peptides coupled to the iscom accompany the iscom complex and settle at an S-value of approximately 19 S, i.e. the same S-value as that of the iscom itself.

Example 32. Preparation of iscoms with malaria peptide, an octapeptide (Glu - Glu - Asu - Val - Glu - His - Asp - Ala). The primary iscom was prepared as described in Example 25 with the dialysis method with the envelope proteins from the influenza virus strain PR 8.

* One ml PR 8 iscom in PBS (0.98 mg/ml) was mixed with 1.56 mg malaria/peptide;
* Ten µl 25% glutaraldehyde was added to the final concentration of 0.25%;
* Incubation was done at 24 hrs at room temperature under slow stirring;
* The preparation was dialyzed for 24 hrs at 4° C against PBS.

The total protein content was estimated at 1.02 mg/ml according to Bradford (Analyt Biochem 1976, pp 248-254).

About 25% of the peptide was calculated to bind to the PR 8 iscom. Typical iscoms were seen in electron microscopy.

Five mice were inoculated subcutaneously with 50 µg of the conjugate. After two weeks blood was collected for preparation of serum. At the same time a second dose of 50 µg conjugate was given. After a further week the mice were bled again and serum prepared.

The serum antibody response was tested in an ELISA test using pooled sera from the mice.

The ELISA was performed in plastic trays (Nunc 96 F 2 - 69620 PS SH).

The coating was performed with the peptide conjugated to bovine serum albumin (BSA) - (one mg BSA and 1 mg peptide using glutaraldehyde as described above and at a final concentration of 0.25%). The mixture was dialyzed as described above. One µg of the conjugate (BSA-peptide) per ml in the coating buffer, i.e. 0.05 M carbonate buffer pH 9.6 was used and 100 µl was added to each well in the tray. Incubation was performed at 4° C over night.

After two washings with phosphate buffer containing 0.05% Tween 80, the test sera at different dilutions were added. Incubation was performed for 1 hour at room temperature. After washings as described above a horse-radish peroxidase labelled rabbit anti-mouse serum (Dakopatts, Copenhagen) was added. The incubation was done for 1 hour at room temperature. After that the substrate Trimethylbenzidine was added.

The reaction was stopped after 10 minutes and the reading was done at 405 nm.

Results: Two weeks after the first vaccination the pooled sera showed a dilution titer of 1:300. One week after the second vaccination the titer has risen to 1:700.

None of the mice show any side effects due to immunization. The conventional way to immunize with peptides includes coupling of the peptide to a protein, e.g. BSA or KLH. That conjugate is then mixed or emulgated with an oil adjuvant, e.g. Freund incomplete or Freund's complete adjuvant, which gives severe side effects in the form of local or systemic reactions. These types of adjuvants are unacceptable for animal as well as human vaccines.

Example 33. Preparation of Lutenizing Hormon Releasing Hormon (LHRH) a decapeptide (Glu - His - Trp - Ser - Tyr - Gly - Leu - Arg - Pro - Gly). The primary iscom was prepared as described in Example 25 with the dialysis method, with the envelope proteins from the influenza virus strain PR 8.

In the group A (see Table 1) the coupling was done with 1 ml PR 8 iscom in PBS (1 mg/ml) and 1 mg peptide in a one-step procedure as in Example 32.

In the group B, C, D and E (see Table 2) the coupling was done with a two-step procedure. To 1 mg PR 8 iscoms in 1 ml PBS 1.25% glutaraldehyde in $H_2O$ was added to a final concentration of 0.25% and the mixture was dialyzed over night at room temperature against 0.9% NaCl. One mg peptide in 100 $\mu$l 0.9% NaCl was added. After mixing 50 $\mu$l 1 M carbonate buffer pH 9.5 was added and the mixture was incubated at 4°C for 24 hrs. After that the mixture was dialyzed against PBS for 24 hrs.

The protein content was determined with the method of Bradford and diluted to 1 mg/ml.

About 25% of the peptide was calculated to bind to the PR 8 iscom. The typical iscom structure was seen in electron microscopy.

Mice were immunized twice subcutaneously four weeks apart with doses ranging from 0.1 $\mu$g to 3 $\mu$g per dose as shown in Table 1. Blood samples were collected at the time for immunizations and four weeks after the second immunization.

The immune response was measured in ELISA as described in Example 32 using peptide conjugated to BSA as test-antigen.

The titer is expressed as dilution of the mouse serum which gives a positive reading at 405 nm.

The results are given in Table 1. All mice responded with serum antibody titres. After one immunization 3 out

Table 1                                    ELISA TITRE DILUTION

| PEPTIDE | GROUP DOSE | 1st IMMUNIZATION | 2nd IMMUNIZATION |
|---------|------------|------------------|------------------|
| LHRH | B  0.1 µg | <100<br>112<br><100<br><100<br><100 | 157<br>149<br>200<br>190<br>126 |
| LHRH | C  0.3 µg | <100<br>Mean 5-pooled<br>sera | 292<br>162<br>112<br>100<br>114 |
| LHRH | D  1.0 µg | 112<br>103<br><100<br>128<br><100 | 283<br>209<br>274<br>240<br>419 |
| LHRH | E  3.0 µg | 134<br><100<br><100 | 274<br>>600<br>>600 |
| LHRH | A  1.0 µg | 248<br>100<br><100 | >600<br>522<br>600 |

Table 2

ELISA TITRE DILUTION

| PEPTIDE | GROUP DOSE | 1st IMMUNIZATION | 2nd IMMUNIZATION |
|---------|------------|------------------|------------------|
| FMD | A  1.0 µg | <100<br><100<br><100<br><100<br><100 | <100<br><100<br><100<br>100<br>196 |
| FMD | B  3.0 µg | 0<br>0.26<br>0.10 | 400<br>226<br>163 |

of 5 mice immunized with 1 µg peptide or more had developed detectable antibody to LHRH.

After two immunizations all mice responded with increased antibody titres. The mice immunized with 0.1 µg had also responded with a good antibody response.

No side effects e.g. as local reaction or systemic reactions were observed either after one or two immunizations.

Example 34. Preparation of iscoms with foot-and-mouth disease (FMD) peptide (16 amino acids (Leu - Arg - Gly - Asp - Leu - Gly - Val - Leu - Ala - Glu - Lys - Val - Ala - Arg - Tyr - Leu). The primary iscom was prepared with the envelope proteins of the influenza virus strains PR 8 as described in Example 25.

20

The coupling was done with a two step procedure with glutaraldehyde as described in Example 33 using 1 mg PR 8 iscoms and 1 mg peptide.

The protein content after coupling was determined with the method of Brandford and diluted to 1 mg/ml. About 25% of the peptide was calculated to bind to the PR 8 iscoms.

In electron microscopy the typical morphology of iscoms were found.

Mice were immunized twice subcutaneously four weeks apart as shown in Table 2 with one or two $\mu$g of peptide conjugated to a PR 8 iscom.

The antibody response of the mice were measured on the mouse sera with ELISA as described in Example 32 and 33 using the FMD peptide conjugated to BSA as test antigen. The titre is expressed as the dilution of the mouse serum which gives a positive reading at 405 nm.

The results are shown in Table 2. The mice did not respond with detectable serum antibody after one immunization when the antibody response was measured in serum two and four weeks after immunization. Four weeks after the second immunization serum from the immunized mice were tested and all mice had responded with serum antibody to the peptide.

None of the mice showed side effects due to the immunization either as local reactions or systemic reactions.

Example 35. Preparation of HTLV-III iscoms enriched with gp 120. Purified viruses of the family of retroviruses generally loose the external part of the envelope protein. That external part is crucial for induction of protective immunity. This external part can be recovered from the tissue culture fluid obtained from the cultures of virus and from fluid obtained during the purification of virus e.g. supernatant after ultracentrifugation of virus.

10 mg HTLV-III virus in 1 ml PBS is solubilized with 1% N-decanyl-N-methyl glucamin. The mixture is incubated for 1 hour at room temperature.

The solubilized membrane proteins are separated from the nucleic acid and attached proteins by centrifugation through sucrose e.g. 20% containing the same detergent e.g. 0.5% and 0.1% Quil A in PBS:

The fractions of sucrose, detergent and Quil A containing membrane proteins are dialyzed against 0.05 M ammonium acetate (the buffer not critical) the first 6 hrs at room temperature and thereafter at $+4^{\circ}$ C.

0.3 mg of gp 120 in 1 ml enriched from tissue culture fluid by affinity chromatography with lens lectin or by anti-gp 120 attached to sepharose is incubated with glutaraldehyde final concentration 0.25% over night at room temperature. After that it is dialyzed against 0.9% NaCl.

The HTLV-III iscoms prepared above in a volume of 2 ml were added and 150 $\mu$l 1 M carbonate buffer pH 9.5. The preparation was incubated for 24 hrs at $4^{\circ}$ C and thereafter dialyzed against PBS for 24 hrs at $4^{\circ}$ C.

The typical formation of iscoms was seen in electron microscopy.

Example 36. Three sequences hybride DNA product from FeLV gp 70 were conjugated to stearyl amine incorporated into liposomes with the glutaraldehyde two-step method (see Example 33). 10 mg (10 mg/ml) liposomes in PBS pH 7 was activated with a final concentration of 1.25% glutardialdehyde at room temperature over night. Excess glutardialdehyde was removed by either dialysis or gel filtration.

3 mg activated liposomes were mixed with 1 mg of each FeLV gp 70 polypeptide, the volume was adjusted to 1 ml and the pH was raised by adding 100 $\mu$l 1 M NaCO$_3$ pH 9.6. The mixture was incubated over night, and purified from unbound polypeptide by gel filtration (e.g. S-300).

The polypeptide-fatty acid was extracted with 2% N-decanoyl-N-methylglucamine (MEGA-10) and separated from excess lipid by centrifugation on a sucrose gradient (5-30% sucrose) containing 0.3% MEGA-10.

The polypeptide was collected, Quil A was added to a final concentration of 0.1% and the mixture was extensively dialyzed against PBS, the first 4-6 hrs at room temperature, then at $+4^{\circ}$ C.

## Claims

1. A multivalent immunogenic complex, characterized in that it consists of a carrier molecule, which carrier molecule contains first antigenic protein(s) or peptide(s) with hydrophobic regions and at least one glycoside, which complex has an open spherical structure consisting of circular subunits or parts of the spheric structure (see Figs. 2 and 3) excluding liposomes, to said carrier molecule being bound a second antigenic molecule or molecules selected from peptides, proteins carbohydrates, lipoproteins, glycolipides or small molecules, such as biotine, by coupling with known methods between functional coupling groups in the bound molecules and functional groups in the carrier molecule.

2. An immunogenic ccmplex according to Claim 1, characterized in that the molecules coupled to the carrier are selected from peptides having 1-40, particularly 10-25 amino acids, whereby when the number of amino acids is ≤10, the peptides may be coupled to aliphatic chains having 2-12 carbon atoms and 6-26 OH-groups or peptides having 1-20 amino acids, preferably hydrophilic amino acids, via functional groups, particularly having amino-acid sequences representative for Malaria, Polio, particularly 277-300, peptides obtained from Hepatite-B virus, particularly peptides in the regions 32-74, 110-156, and particularly peptides containing the amino-acid sequences 144-145, Rabies virus, particularly peptides in the regions 1-50, 290-320 and particularly in the region 100-175, Influenza virus peptides containing the amino-acid sequence 140-146, 187-196 or selected in the region Cys 52 - Cys 278 or 207-220, and foot-and-mouth disease virus, particularly the foot-and-mouth disease virus peptides, 141-160, 144-160, 146-154, 144-150, 142-158, growth factors for T-cells, preferably peptides having the amino-acid sequences 79-92, 139-153C, 111-125 and 18-32C, peptides obtained from Epstein Barr virus, particularly the sequences A: Asp, Val, Gly, Gly, Lys, Lys, His, Gln, Lev, Asp, Cys, Leu, Leu; B: His, His, Ala, Glu, Asn, Gln, Asn, Pro, Cys, Leu, Leu; C: Ala, Trp, Pro, Asn, Asn, Thr, Glu, Thr, Asp, Phe, Lys, Cys, Leu, Leu, antigenic determinants from HTLV 1, 2 or 3 virus,peptides in blood group substances; peptides, polypeptides and larger proteins, particularly steroid hormones, peptide hormones and prostaglandin hormones, such as tyrotropin, adenocorticotropin, luteinizing hormones, gonadotropin releasing hormone, follicle stimulating hormones, prolactin, growth hormones, oxytocin, vasopressin, paratyroidea hormone, calcitonin, insulin, glucagon, and enzymes such as lysozyme and peroxidase; carbohydrates and carbohydrate-containing structures, such as lipopolysaccharides, polysaccharides from microorganisms with capsules, such as E.coli, particularly K-antigenes 1-13, Haemophilus influenza, meningococcus, the oligosaccharide portion of glycoproteins in blood group substances in gangliosides, such as GM1, and glyomgangliosides.

3. Immunogenic complex according to Claims 1 or 2, characterized in that the carrier has been obtained by selecting the membrane proteins from enveloped virus, particularly Orthomyxo-viridae, Paramyxo-viridae, Retro-viridae, Rabdo-viridae, Toga-viridae, Herpes-viridae and Hepatite-B-virus, membrane proteins from toxoplasma, non-enveloped Picorna-viridae, Parvo-viridae, Reo-viridae,and the glycoside has been selected from saponines such as glycoside extract from, for example, Quillaja sapnaria molina, Aesculus hippocastanum or Gypophilla struthim, preferably DQ, Quil A, asceine, sapoalbin.

4. Immunogenic complex according to any one of Claims 1-3, characterized in that the carrier has been obtained by selecting the proteins and the peptides from hydrophobic membrane proteins or non-membrane proteins and non-hydrophobic proteins from viruses, mycoplasmas, bacteria, parasites, animal cells, said non-hydrophobic proteins having been made hydrophobic by coupling thereto hydrophobic groups selected from aliphatic groups, hydrophobic peptides and other hydrophobic structures, for example steroids such as cholic acid and cholesterol derivatives, amphiphatic proteins or peptides containing unavailable hydrophobic groups which have been made available by mild denaturization; or from synthetic or proteins or peptides produced by hybrid DNA technique.

5. Immunogenic complex according to Claims 1-4, characterized in that the carrier is obtained by viruses, mycoplasmas, bacterias, parasites, animal cells or hydrophobic peptides or proteins being mixed with the solubilizing agent chosen among an ionic, non-ionic, Zwitterionic or gallic acid detergent, alcohols, small amphiphatic molecules, water soluble peptides or proteins or mixtures thereof in buffered, possibly saline, solution, the mixture being layered on top of a solution containing solubilizing agent, which lies in turn over a gradient containing glycoside and is centrifuged at least at 100,000 g, the proteinaceous fraction being isolated, dialyzed against buffer solution or by the microorganisms, cells, proteins or peptides, after they have been mixed with the solubilizing agent in buffered saline solution being reacted with glycoside and dialyzed against buffer preferably ammonium acetate or layered directly on a gradient and centrifuged at least at 100,000 g, whereafter the protein-containing top fraction is collected, reacted with glycoside and dialyzed against buffer, preferably ammonium acetate or by the mixture of microorganisms, animal cells, proteins or peptides and solubilizing agent in the buffer or the proteinaceous top fraction obtained when the mixture of microorganisms, cells, proteins or peptides and solubilizing agent in buffered saline solution is centrifuged through a gradient, being separated by electrophoresis or chromatographically from the solubilizing agent and collected in a solution containing the glycoside, whereafter the protein complex obtained is possibly concentrated, e.g. by lyophilisation, vacuum dialysis or ultracentrifuging or is purified further by gradient centrifuging.

6. A method of preparing an immunogenic complex according to any one of Claims 1-5, characterized in that a carrier molecule is prepared by mixing viruses, mycoplasmas, bacterias, parasites, animal cells, proteins or peptides having hydrophobic regions with one or more solubilizing agents to form a complex between proteins or peptides and solubilizing agent; whereafter the proteins or peptides are separated from the solubilizing agent in the presence of a glycoside solution containing one or more glycosides having hydrophobic and hydrophilic regions in a concentration of at least the critical micellular concentration, or alternatively by separating said charged monomeric antigenic proteins or peptides from said solubilizing agent and transferring them directly to said glycoside solution, whereby a protein complex with glycoside is formed, which complex is isolated and purified, whereafter the carrier molecule is bound to molecules selected from peptides, proteins, carbohydrates, lipoproteins, glycolipides or biotine by coupling between functional coupling groups in the molecules bound to the carrier and functional groups in the carrier molecules.

7. A method according to Claim 6, characterized by reacting approximately 1 mg, preferably 250-500 $\mu$g of the molecule to be coupled with approximately 1 mg (measured as protein content) of the carrier molecule at coupling between the functional groups.

8. An immuno-stimulating composition, characterized in that it contains as the active substance at least one immunogenic complex according to any one of Claims 1-6, optionally in mixture with pharmaceutically acceptable additives.

9. Vaccine, characterized in that it contains at least one immunogenic complex according to any one of Claims 1-6 as active substance.

10. Reagens, characterized in that it comprises at least one immunogenic complex according to any one of Claims 1-5.

## Claims for the following Contracting State: AT

1. A process for preparing an immunogenic complex, characterized in that a carrier molecule is prepared by mixing viruses, mycoplasmas, bacterias, parasites, animal cells, proteins or peptides having hydrophobic regions with one or more solubilizing agents to form a complex between proteins or peptides and solubilizing agent; whereafter the proteins or peptides are separated from the solubilizing agent in the presence of a glycoside solution containing one or more glycosides having hydrophobic and hydrophilic regions in a concentration of at least the critical micellular concentration, or alternatively by separating said proteins or peptides from said solubilizing agent and transferring them directly to said glycoside solution, whereby a protein complex with glycoside is formed, whereafter the carrier molecule is bound to one or more molecules selected from peptides, proteins, carbohydrates, lipoproteins, glycolipides or biotine by coupling between functional coupling groups in the molecules bound to the carrier and functional groups in the carrier molecules.

2. A process according to Claim 1, characterized in that the molecules coupled to the carrier are selected from peptides having 1-40, particularly 10-25 amino acids, whereby when the number of amino acids is 10, the peptides may be coupled to aliphatic chains having 2-12 carbon atoms and 6-26 OH-groups or peptides having 1-20 amino acids, preferably hydrophilic amino acids, via functional groups, particularly having amino-acid sequences representative for Malaria, Polio, particularly 277-300, peptides obtained from Hepatite-B virus, particularly peptides in the regions 32-74, 110-156, and particularly peptides containing the amino-acid sequences 144-145, Rabies virus, particularly peptides in the regions 1-50, 290-320 and particularly in the region 100-175, Influenza virus peptides containing the amino-acid sequence 140-146, 187-196 or selected in the region Cys 52 - Cys 278 or 207-220, and foot-and-mouth disease virus, particularly the foot-and-mouth disease virus peptides, 141-160, 144-160, 146-154, 144-150, 142-158, growth factors for T-cells, preferably peptides having the amino-acid sequences 79-92, 139-153C, 111-125 and 18-32C, peptides obtained from Epstein Barr virus, particularly the sequences A: Asp, Val, Gly, Gly, Lys, Lys, His, Gln, Lev, Asp, Cys, Leu, Leu; B: His, His, Ala, Glu, Asn, Gln, Asn, Pro, Cys, Leu, Leu; C: Ala, Trp, Pro, Asn, Asn, Thr, Glu, Thr, Asp, Phe, Lys, Cys, Leu, Leu, antigenic determinants from HTLV 1, 2 or 3 virus peptides in blood group substances; peptides, polypeptides, and larger proteins, particularly steroid hormones, peptide hormones and prostaglandin hormones, such as tyrotropin, adenocorticotropin, luteinizing hormones, gonadotropin releasing hormone, follicle-stimulating hormones, prolactin, growth hormones, oxytocin, vasopressin, paratyroidea hormone, calcitonin, insulin, glucagon, and enzymes such as lysosyme and peroxidase; carbohydrates and carbohydrate-containing structures, such as lipopoly-saccharides, polysaccharides from microorganisms with capsules, such as E.coli, particularly K-antigenes 1-13, Haemophilus influenza, meningococcus, the oligosaccharide portion of glycoproteins in blood-group substances in gangliosides, such as GM1, and glyomgangliosides.

3. A process according to any one of Claims 1-2, characterized in that the carrier has been obtained by selecting the membrane proteins from enveloped virus, particularly Ortomyxoviridae, Paramyxoviridae, Retroviridae, Rabdoviridae, Togaviridae, Herpesviridae and Hepatite-B virus, membrane proteins from toxoplasma, non-enveloped Picornaviridae, Parvoviridae, Reoviridae, and the glycoside has been selected from saponines such as glycoside extract from, for example, Quillaja sapnaria molina, Aesculus hippocastanum or Gypophilla struthim, preferably DQ, Quil A, ascein, sapoalbin.

4. A process according to any one of Claims 1-3, characterized in that the carrier has been obtained by selecting the proteins and the peptides from hydrophobic membrane proteins or non-membrane proteins and non-hydrophobic proteins from viruses, mycoplasmas, bacterias, parasites, animal cells, said non-hydrophobic proteins having been made hydrophobic by coupling thereto hydrophobic groups selected from aliphatic groups, small hydrophobic peptides and other hydrophobic structures, for example steroids such as cholic acid and cholesterol derivatives, amphiphatic proteins or peptides containing unavailable hydrophobic groups which have been made available by mild denaturization; or from synthetic or proteins or peptides produced by hybrid DNA technique.

5. A process according to Claims 1-4, characterized in that the carrier is obtained by viruses, mycoplasmas, bacterias, parasites, animal cells or hydrophobic peptides or proteins being mixed with the solubilizing agent chosen among an ionic, non-ionic, Zwitterionic or gallic acid detergent, alcohols, small amphiphatic molecules, water soluble peptides or proteins or mixtures thereof in buffered, possibly saline, solution, the mixture being layered on top of a solution containing solubilizing agent, which lies in turn over a gradient containing glycoside and is centrifuged at least at 100,000 g, the proteinaceous fraction being isolated, dialyzed against buffer solution or by the microorganisms, animal cells, proteins or peptides, after they have been mixed with the solubilizing agent in buffered saline solution being reacted with glycoside and dialyzed against buffer, preferably ammonium acetate buffer, or layered directly on a gradient and centrifuged at least at 100,000 g, whereafter the protein-containing top fraction is collected, reacted with glycoside and dialyzed against buffer, preferably ammonium acetate or by the mixture of microorganisms, animal cells, proteins or peptides and solubilizing agent in the buffer or the proteinaceous top fraction obtained when the mixture of micro-organisms, animal cells, proteins or peptides and solubilizing agent in buffered saline solution is centrifuged through a gradient, being separated by electrophoresis or chromatographically from the solubilizing agent and collected in a solution containing the glycoside, whereafter the protein complex obtained is possibly concentrated, e.g. by lyophilisation, vacuum dialysis or ultracentrifuging or is purified further by gradient centrifuging.

6. A process according to Claim 5, characterized by reacting approximately 1 mg, preferably 250-500 $\mu$g of the molecule to be coupled with approximately 1 mg (measured as protein content) of the carrier molecule at coupling between the functional groups.

7. A process for preparing an immunostimulating composition, characterized in that at least one immunogenic complex is prepared according to any one of Claims 1-6, and optionally is mixed with pharmaceutically acceptable additives.

8. A process for preparing a vaccine, characterized in that at least one immunogenic complex is prepared according to any one of Claims 1-6.

9. A reagent, characterized in that it comprises at least one immunogenic complex according to any one of Claims 1-6.

## Revendications

1. Complexe immunogène multivalent, caractérisé en ce qu'il est constitué d'une molécule vectrice, laquelle molécule vectrice contient une ou plusieurs premières protéines antigéniques ou un ou plusieurs premiers peptides antigéniques avec des régions hydrophobes et au moins un glycoside, lequel complexe a une structure sphérique ouverte constituée de sous-unités ou parties circulaires de la structure sphérique (voir les figures 2 et 3) à l'exclusion des liposomes, une ou plusieurs secondes molécules antigéniques, choisies parmi les peptides, les protéines, les glucides, les lipoprotéines, les glycolipides ou les petites molécules, telles que la biotine, étant liées à ladite molécule vectrice par couplage selon des procédés connus entre des groupes fonctionnels de couplage dans les molécules liées et des groupes fonctionnels dans la molécule vectrice.

2. Complexe immunogène selon la revendication 1, caractérisé en ce que les molécules couplées au vecteur sont choisies parmi les peptides ayant 1 à 40 et en particulier 10 à 25 amino-acides, si bien que, lorsque le nombre des amino-acides est < 10, les peptides peuvent être couplés à des chaînes aliphatiques ayant 2 à 12 atomes de carbone et 6 à 26 groupes OH, ou à des peptides ayant 1 à 20 amino-acides, de préférence des amino-acides hydrophiles, via des groupes fonctionnels, en particulier ceux ayant des séquences d'amino-acides représentatives du paludisme, les peptides dérivés du poliovirus, en particulier 277-300, les peptides obtenus à partir du virus de l'hépatite B, en particulier les peptides des régions 32-74, 110-156, et particulièrement les peptides contenant les séquences d'amino-acides 144-145, le virus de la rage, en particulier les peptides des régions 1-50, 290-320 et en particulier de la région 100-175, les peptides du virus grippal contenant les séquences d'amino-acides 140-146, 187-196 ou choisies dans la région Cys 52 - Cys 278 ou 207-220, et le virus de la fièvre aphteuse, en particulier les peptides du virus de la fièvre aphteuse 141-160, 144-160, 146-154, 144-150, 142-158, les facteurs de croissance des cellules T, de préférence les peptides ayant les séquences d'amino-acides 79-92, 139-153C, 111-125 et 18-32C, les peptides obtenus à partir du virus d'Epstein-Barr, en particulier les séquences A : Asp, Val, Gly, Gly, Lys, Lys, His, Gln, Leu, Asp, Cys, Leu, Leu ; B : His, His, Ala, Glu, Asn, Gln, Asn, Pro, Cys, Leu, Leu ; C : Ala, Trp, Pro, Asn, Asn, Thr, Glu, Thr, Asp, Phe, Lys, Cys, Leu, Leu, les déterminants antigéniques des virus HTLV 1, 2 ou 3, les peptides des substances des groupes sanguins ; les peptides, les polypeptides et les protéines plus grosses, en particulier les hormones stéroïdiennes, les hormones peptidiques et les hormones de type prostaglandine, telles que la thyréotrophine, l'hormone adénocorticotrope, les hormones lutéinisantes, la gonadolibérine, la folliculostimuline, la prolactine, les hormones de croissance, l'ocytocine, la vasopressine, l'hormone parathyroïdienne, la calcitonine, l'insuline, le glucagon et des enzymes telles que le lysozyme et la peroxydase ; des glucides et des structures contenant un glucide, telles que les lipopolysaccharides, les polysaccharides des microorganismes ayant des capsules, tels que E. coli, en particulier les antigènes K 1-13, Haemophilus influenzae, le méningocoque, la portion oligosaccharidique des glycoprotéines des substances des groupes sanguins, des gangliosides, tels que GM1, et des gliomegangliosides.

3. Complexe immunogène selon la revendication 1 ou 2, caractérisé en ce que le vecteur a été obtenu par sélection des protéines de membrane de virus à enveloppe, en particulier d'Orthomyxovirus, de Paramyxovirus, de Rétrovirus, de Rabdovirus, de Togavirus, d'Herpèsvirus et de virus de l'hépatite B, de protéines de membrane de toxoplasmes, de Picornavirus, Parvovirus et Réovirus sans enveloppe, et le glucoside a été choisi parmi les saponines telles que l'extrait glycosidique par exemple de Quillaja saponaria Molina, Aesculus hippocastanum ou Gypophilla struthium, de préférence DQ, Quil A, l'ascéine et la sapoalbine.

4. Complexe immunogène selon l'une quelconque des revendications 1 à 3, caractérisé en ce que le vecteur a été obtenu par sélection des protéines et des peptides à partir des protéines membranaires ou des protéines non membranaires hydrophobes et des protéines non hydrophobes de virus, de mycoplasmes, de bactéries, de parasites, de cellules animales, lesdites protéines non hydrophobes ayant été rendues hydrophobes par couplage de groupes hydrophobes choisis parmi les groupes aliphatiques, les peptides hydrophobes et d'autres structures hydrophobes, par exemple des stéroïdes, tels que l'acide cholique et les dérivés du cholestérol, des protéines ou peptides amphiphatiques contenant des groupes hydrophobes non disponibles qui ont été rendus disponibles par dénaturation modérée ; ou à partir de protéines ou de peptides synthétiques ou produits selon une technique à ADN hybride.

5. Complexe immunogène selon les revendications 1 à 4, caractérisé en ce que le véhicule est obtenu par mélange de virus, de mycoplasmes, de bactéries, de parasites, de cellules animales ou de peptides ou protéines hydrophobes, avec l'agent solubilisant choisi parmi des détergents anioniques, non ioniques, zwitterioniques ou de type acide gallique, les alcools, les petites molécules amphiphatiques, les peptides ou protéines solubles dans l'eau ou leurs mélanges dans une solution tamponnée éventuellement salée, le mélange étant déposé sur une solution contenant un agent solubilisant, qui elle-même est placée sur un gradient contenant un glycoside et est centrifugée à au moins 100 000 g, la fraction protéinique étant isolée, dialysée contre une solution tampon, ou par réaction des microorganismes, des cellules, des protéines ou des peptides, après qu'ils aient été mélangés avec l'agent solubilisant dans une solution salée tamponnée avec un glycoside et dialyse contre un tampon, de préférence à l'acétate d'ammonium, ou déposés directement sur un gradient et centrifugés à au moins 100 000 g, après quoi la fraction supérieure contenant les protéines est recueillie, mise à réagir avec un glycoside et dialysée contre un tampon, de préférence à l'acétate d'ammonium, ou par séparation par électrophorèse ou chromatographie d'avec l'agent solubilisant du mélange des microorganismes, des cellules animales, des protéines ou des peptides et d'un agent solubilisant dans la fraction supérieure de tampon ou protéinique obtenue lorsque le mélange des microorganismes, des cellules, des protéines ou des peptides et de l'agent solubilisant dans une solution salée tamponnée est centrifugé à travers un gradient et recueilli dans une solution contenant le glycoside, après quoi le complexe protéinique obtenu est éventuellement concentré par exemple par lyophilisation, dialyse sous vide ou ultracentrifugation ou est purifié encore par centrifugation sur gradient.

6. Procédé de préparation d'un complexe immunogène selon l'une quelconque des revendications 1 à 5, caractérisé en ce qu'une molécule vectrice est préparée par mélange de virus, de mycoplasmes, de bactéries, de parasites, de cellules animales, de protéines ou de peptides ayant des régions hydrophobes, avec un ou plusieurs agents solubilisants pour former un complexe entre les protéines ou peptides et l'agent solubilisant ; après quoi, les protéines ou peptides sont séparés d'avec l'agent solubilisant en présence d'une solution glycosidique contenant un ou plusieurs glycosides ayant des régions hydrophobes et hydrophiles à une concentration qui est au moins la concentration micellaire critique, ou sinon par séparation desdites protéines ou desdits peptides antigéniques monomères chargés d'avec l'agent solubilisant et transfert de ceux-ci directement dans ladite solution glycosidique, pour former un complexe protéinique avec le glycoside, lequel complexe est isolé et purifié, après quoi la molécule vectrice est liée, à des molécules choisies parmi les peptides, les protéines, les glucides, les lipoprotéines, les glycolipides ou la biotine, par couplage entre les groupes de couplage fonctionnels des molécules fixées au vecteur et les groupes fonctionnels des molécules vectrices.

7. Procédé selon la revendication 6, caractérisé par la réaction d'environ 1 mg, de préférence 250 à 500 $\mu$g, de la molécule à coupler avec environ 1 mg (mesuré en teneur en protéines) de la molécule vectrice lors du couplage entre les groupes fonctionnels.

8. Composition immunostimulante, caractérisée en ce qu'elle contient comme substance active au moins un complexe immunogène selon l'une quelconque des revendications 1 à 6, le cas échéant en mélange avec des additifs pharmaceutiquement acceptables.

9. Vaccin, caractérisé en ce qu'il contient au moins un complexe immunogène selon l'une quelconque des revendications 1 à 6 comme substance active.

10. Réactif, caractérisé en ce qu'il comprend au moins un complexe immunogène selon l'une quelconque des revendications 1 à 5.

**Revendications pour l'Etat contractant suivant: AT**

1. Procédé pour préparer un complexe immunogène, caractérisé en ce qu'une molécule vectrice est préparée par mélange de virus, de mycoplasmes, de bactéries, de parasites, de cellules animales, de protéines ou de peptides ayant des régions hydrophobes, avec un ou plusieurs agents solubilisants pour former un complexe entre les protéines ou peptides et l'agent solubilisant ; après quoi, les protéines ou peptides sont séparés d'avec l'agent solubilisant en présence d'une solution glycosidique contenant un ou plusieurs glycosides ayant des régions hydrophobes et hydrophiles à une concentration qui est au moins la concentration micellaire critique, ou sinon par séparation desdites protéines ou desdits peptides d'avec l'agent solubilisant et transfert de ceux-ci directement dans ladite solution glycosidique, pour former un complexe protéinique avec le glycoside, après quoi la molécule vectrice est liée, à des molécules choisies parmi les peptides, les protéines, les glucides, les lipoprotéines, les

EP 0 180 564 B1

glycolipides ou la biotine, par couplage entre les groupes de couplage fonctionnels des molécules fixées au vecteur et les groupes fonctionnels des molécules vectrices.

2. Procédé selon la revendication 1, caractérisé en ce que les molécules couplées au vecteur sont choisies parmi les peptides ayant 1 à 40 et en particulier 10 à 25 amino-acides, si bien que, lorsque le nombre des amino-acides est < 10, les peptides peuvent être couplés à des chaînes aliphatiques ayant 2 à 12 atomes de carbone et 6 à 26 groupes OH, ou à des peptides ayant 1 à 20 amino-acides, de préférence des amino-acides hydrophiles, via des groupes fonctionnels, en particulier ceux ayant des séquences d'amino-acides représentatives du paludisme, les peptides dérivés du poliovirus, en particulier 277-300, les peptides obtenus à partir du virus de l'hépatite B, en particulier les peptides des régions 32-74, 110-156, et particulièrement les peptides contenant les séquences d'amino-acides 144-145, le virus de la rage, en particulier les peptides des régions 1-50, 290-320 et en particulier de la région 100-175, les peptides du virus grippal contenant les séquences d'amino-acides 140-146, 187-196 ou choisies dans la région Cys 52 - Cys 278 ou 207-220, et le virus de la fièvre aphteuse, en particulier les peptides du virus de la fièvre aphteuse 141-160, 144-160, 146-154, 144-150, 142-158, les facteurs de croissance des cellules T, de préférence les peptides ayant les séquences d'amino-acides 79-92, 139-153C, 111-125 et 18-32C, les peptides obtenus à partir du virus d'Epstein-Barr, en particulier les séquences A : Asp, Val, Gly, Gly, Lys, Lys, His, Gln, Leu, Asp, Cys, Leu, Leu ; B : His, His, Ala, Glu, Asn, Gln, Asn, Pro, Cys, Leu, Leu ; C : Ala, Trp, Pro, Asn, Asn, Thr, Glu, Thr, Asp, Phe, Lys, Cys, Leu, Leu, les déterminants antigéniques des virus HTLV 1, 2 ou 3, les peptides des substances des groupes sanguins ; les peptides, les polypeptides et les protéines plus grosses, en particulier les hormones stéroïdiennes, les hormones peptidiques et les hormones de type prostaglandine, telles que la thyréotrophine, l'hormone adénocorticotrope, les hormones lutéinisantes, la gonadolibérine, la folliculostimuline, la prolactine, les hormones de croissance, l'ocytocine, la vasopressine, l'hormone parathyroïdienne, la calcitonine, l'insuline, le glucagon et des enzymes telles que le lysozyme et la peroxydase ; des glucides et des structures contenant un glucide, telles que les lipopolysaccharides, les polysaccharides des microorganismes ayant des capsules, tels que E. coli, en particulier les antigènes K 1-13, Haemophilus influenzae, le méningocoque, la portion oligosaccharidique des glycoprotéines des substances des groupes sanguins, des gangliosides, tels que GM1, et des gliomegangliosides.

3. Procédé selon l'une des revendications 1 ou 2, caractérisé en ce que le vecteur a été obtenu par sélection des protéines de membrane de virus à enveloppe, en particulier d'Orthomyxovirus, de Paramyxovirus, de Rétrovirus, de Rabdovirus, de Togavirus, d'Herpèsvirus et de virus de l'hépatite B, de protéines de membrane de toxoplasmes, de Picornavirus, Parvovirus et Réovirus sans enveloppe, et le glucoside a été choisi parmi les saponines telles que l'extrait glycosidique par exemple de Quillaja saponaria Molina, Aesculus hippocastanum ou Gypophilla struthium, de préférence DQ, Quil A, l'ascéine et la sapoalbine.

4. Procédé selon l'une quelconque des revendications 1 à 3, caractérisé en ce que le vecteur a été obtenu par sélection des protéines et des peptides à partir des protéines membranaires ou des protéines non membranaires hydrophobes et des protéines non hydrophobes de virus, de mycoplasmes, de bactéries, de parasites, de cellules animales, lesdites protéines non hydrophobes ayant été rendues hydrophobes par couplage de groupes hydrophobes choisis parmi les groupes aliphatiques, les petits peptides hydrophobes et d'autres structures hydrophobes, par exemple des stéroïdes, tels que l'acide cholique et les dérivés du cholestérol, des protéines ou peptides amphiphatiques contenant des groupes hydrophobes non disponibles qui ont été rendus disponibles par dénaturation modérée ; ou à partir de protéines ou de peptides synthétiques ou produits selon une technique à ADN hybride.

5. Procédé selon l'une quelconque des revendications 1 à 4, caractérisé en ce que le véhicule est obtenu par mélange de virus, de mycoplasmes, de bactéries, de parasites, de cellules animales ou de peptides ou protéines hydrophobes, avec l'agent solubilisant choisi parmi des détergents anioniques, non ioniques, zwitterioniques ou de type acide gallique, les alcools, les petites molécules amphiphatiques, les peptides ou protéines solubles dans l'eau ou leurs mélanges dans une solution tamponnée éventuellement salée, le mélange étant déposé sur une solution contenant un agent solubilisant, qui elle-même est placée sur un gradient contenant un glycoside et est centrifugée à au moins 100 000 g, la fraction protéinique étant isolée, dialysée contre une solution tampon, ou par réaction des microorganismes, des cellules animales, des protéines ou des peptides, après qu'ils aient été mélangés avec

27

l'agent solubilisant dans une solution salée tamponnée avec un glycoside et dialyse contre un tampon, de préférence à l'acétate d'ammonium, ou déposés directement sur un gradient et centrifugés à au moins 100 000 g, après quoi la fraction supérieure contenant les protéines est recueillie, mise à réagir avec un glycoside et dialysée contre un tampon, de préférence à l'acétate d'ammonium, ou par séparation par électrophorèse ou chromatographie d'avec l'agent solubilisant du mélange des microorganismes, des cellules animales, des protéines ou des peptides et d'un agent solubilisant dans la fraction supérieure de tampon ou protéinique obtenue lorsque le mélange des microorganismes, des cellules, des protéines ou des peptides et de l'agent solubilisant dans une solution salée tamponnée est centrifugé à travers un gradient et recueilli dans une solution contenant le glycoside, après quoi le complexe protéinique obtenu est éventuellement concentré par exemple par lyophilisation, dialyse sous vide ou ultracentrifugation ou est purifié encore par centrifugation sur gradient.

6. Procédé selon la revendication 5, caractérisé par la réaction d'environ 1 mg, de préférence 250 à 500 µg, de la molécule à coupler avec environ 1 mg (mesuré en teneur en protéines) de la molécule vectrice lors du couplage entre les groupes fonctionnels.

7. Procédé pour préparer une composition immunostimulante, caractérisé en ce que l'on prépare au moins un complexe immunogène selon l'une quelconque des revendications 1 à 6 et le cas échéant on le mélange avec des additifs pharmaceutiquement acceptables.

8. Procédé pour préparer un vaccin, caractérisé en ce que l'on prépare au moins un complexe immunogène selon l'une quelconque des revendications 1 à 6.

9. Réactif, caractérisé en ce qu'il comprend au moins un complexe immunogène selon l'une quelconque des revendications 1 à 6.

**Patentansprüche**

1. Mehrwertiger immunogenischer Komplex, dadurch gekennzeichnet, daß er aus einem Trägermolekül besteht, welches Trägermolekül ein oder mehrere antigenische Proteine oder Peptide mit hydrophoben Regionen und wenigstens ein Glycosid enthält, welcher Komplex eine offene sphärische Struktur aufweist, die aus kreisförmigen Untereinheiten oder Teilen der sphärischen Struktur (siehe Fig. 2 und 3) besteht, welche Liposome ausschließt, wobei an das Trägermolekül ein zweites antigenisches Molekül oder Moleküle gebunden sind, die aus Peptiden, Protein-Kohlehydraten, Lipoproteinen, Glycolipiden oder kleinen Molekülen, wie Biotin ausgewählt sind und die Ankoppelung nach bekannten Verfahren zwischen funktionellen Kupplungsgruppen in den angebundenen Molekülen und funktionellen Gruppen im Trägermolekül erfolgt.

2. Immunogenischer Komplex nach Anspruch 1, dadurch gekennzeichnet, daß die an den Träger gekuppelten Moleküle ausgewählt sind aus Peptiden, die 1 - 40, insbesondere 10 - 25 Aminosäuren aufweisen, wobei, wenn die Anzahl der Aminosäuren ≤ 10 ist, die Peptide an aliphatische Ketten mit 2 - 12 Kohlenstoffatomen und 6 - 26 OH-Gruppen oder Peptide, die 1 -20 Aminosäuren, vorzugsweise hydrophile Aminosäuren aufweisen, durch Funktionsgruppen gekuppelt sein können, die ins-besondere Aminosäurensequenzen aufweisen, die für Malaria, Polio, insbesondere 277 - 300 repräsentativ sind, Peptide, die vom Hepatitis-B-Virus erhalten werden, insbesondere Peptide in den Bereichen 32 - 74, 110 - 156 und insbesondere Peptide, welche die Aminosäurensequenz 144 - 145 aufweisen, Rabies-Virus, insbesondere Peptide in den Bereichen 1 - 50, 290 - 320 und insbesondere im Bereich 100 - 175, Influenzaviruspeptide, welche die Aminosäurensequenz 140 -146, 187 - 196 aufweisen oder aus dem Bereich Cys 52 - Cys 278 oder 207 - 220 gewählt sind und Maul- und Klauenseuche-Virus, insbesondere die Maul- und Klauenseuche-Viruspeptide 141 - 160, 144 - 160, 146 - 154, 144 - 150, 142 - 158, Wachstumsfaktoren für T-Zellen, vorzugsweise Peptide, welche die Aminosäurensequenzen 79 - 92, 139 - 153 C, 111 - 125 und 18 - 32 C aufweisen, Peptide, die vom Epstein Barr Virus erhalten werden, insbesondere die Sequenzen A : Asp, Val, Gly, Gly, Lys, Lys, His, Gln, Lev, Asp, Cys, Leu, Leu; B: His, Ala, Glu, Asn, Gln, Asn, Pro, Cys, Leu, Leu; C: Ala, Trp, Pro, Asn, Asn, Thr, Glu, Thr, Asp, Phe, Lys, Cys, Leu, Leu, antigenische Determinatoren von HTLV 1, 2 oder 3 Virus, Peptiden in Blutgruppensubstanzen, Peptiden, Polipeptiden und größere Proteine, insbesondere Steroidhormone, Peptidhormone, wie Prostaglandinhormone, wie Tyrotropin, Adnenocorticoprotein, luteinisierende Hormone, Ganadotropin freisetzende Hormone, follikelstimmulierende Hormone, Prolaktin, Wachstumshormone, Oxytozin, Vasopressin, Paratyroidea Hormone, Calcitonin, Insulin, Glucagon und Enzyme, wie Lysozine und Peroxidase, Kohlehydrate und kohlehydrathältige Strukturen, wie Lipopolysaccharide, Polisaccharide von gekapselten Mikroorganismen, wie E. Coli, insbesondere K-Antigene 1 - 13, Haemophilus Influenza, Meningococcus, den Oligosaccharidanteil von Glycoproteinen in Blutgruppensubstanzen in Gangliosiden, wie GM1, Glyomganglioside.

3. Immunogenischer Komplex nach Anspruch 1 oder, dadurch gekennzeichnet, daß der Träger erhalten wurde durch Auswahl der Membranproteine von umhüllten Viren, insbesondere Orthomyxo-Viridae, Paramyxo-Viridae, Retro-Viridae, Rabto-Viridae, Toga-Viridaer, Herpes-Viridae und Hepatitis-B-Virus, der Membranproteine von Toxoplasma nicht umhüllten Picorn-Viridae, Parvo-Viridae, Reo-Viridae und die Glycoside aus Saponinen ausgewählt wurden, wie Glycosidextrakten von beispielsweise Quillaja Saponaria Molina, Aesculus Hippocastanum oder Gypophilla Struthim, vorzugsweise DQ, Qil A, Ascein, Sapoalbin.

4. Immunogenischer Komplex nach einem der Ansprüche 1 bis 3, dadurch gekennzeichnet, daß der Träger dadurch erhalten wird, daß die Proteine und Peptide von hydrophoben Membranproteinen oder nicht-Membranproteinen und nichthydrophobischen Proteinen von Viren, Mykoplasmen, Bakterien, Parasiten und tierischen Zellen ausgewählt werden und die nicht-hydrophobischen Proteine durch Ankupplung hydrophobischer Gruppen an sie hydrophob gemacht wurden, welche hydrophoben Gruppen aus aliphatischen Gruppen, hydrophoben Peptiden und anderen hydrophoben Strukturen ausgewählt wurden, beispielsweise von Steroiden, wie Gallensäure und Cholesterinderivaten, amphiphatischen Proteinen oder Peptiden, die unzugängliche hydrophobe Gruppen aufweisen, die durch milde Denaturisierung zugänglich gemacht wurden, synthetisch hergestellt sind oder durch Hybrid-DNA-Technik erzeugten Proteinen oder Peptiden.

5. Immunogenischer Komplex nach den Ansprüchen 1 bis 4, dadurch gekennzeichnet, daß der Träger aus Viren, Mykoplasmen, Bakterien, Parasiten, tierischen Zellen oder hydrophoben Peptiden oder Proteinen erhalten wird, die mit einem Lösungsmittel gemischt werden, das aus einem ionischen, nicht-ionischen, zwitter-ionischen oder Gallussäure-Detergent, Alkoholen, kleinen amphiphatischen Molekülen, wasserlöslichen Peptiden oder Proteinen in gepufferter, möglicherweise salziger Lösung ausgewählt ist, die Mischung oben auf eine Lösung aufgelegt wird, die Lösungsmittel enthält und die ihrerseits über einem Gradienten liegt, der Glycoside enthält, wonach bei wenigstens 100 000 g zentrifugiert, die proteinhältige Fraktion isoliert gegen eine Pufferlösung dialysiert oder die Mikroorganismen, Zellen, Proteine oder Peptide nachdem sie mit dem Lösungsmittel in gepufferter Salzlösung gemischt wurden, mit Glycosid reagiert und gegen einen Puffer, vorzugsweise Ammoniumacetat dialysiert oder direkt auf einen Gradienten abgelegt und bei wenigstens 100 000 g zentrifugiert werden, wonach die proteinhältige Oberfraktion gesammelt, mit Glycosid reagiert und gegen einen Puffer, vorzugsweise Ammoniumacetat dialysiert wird oder die Mischung aus Mikroorganismen, tierischen Zellen, Proteinen oder Peptiden und Lösungsmittel im Puffer oder die proteinhältige Oberfraktion, die erhalten wird, wenn die Mischung aus Mikroorganismen, Zellen, Proteinen oder Peptiden und Verflüssigungsmittel in gepufferter salziger Lösung durch einen Gradienten zentrifugiert wird, durch Elektrophorese oder auf chromatographischen Wege vom Lösungsmittel getrennt und in einer Lösung gesammelt wird, welche das Glycosid enthält, wonach der erhaltene Proteinkomplex gegebenenfalls z. B. durch Lyophilisierung, Vakuumdialyse oder Ultrazentrifugierung konzentriert oder durch Gradientenzentrifugierung weiter gereinigt wird.

6. Verfahren zur Herstellung eines immunogenischen Komplexes nach einem der Ansprüche 1 bis 5, dadurch gekennzeichnet, daß ein Trägermolekül dadurch hergestellt wird, daß Viren, Mykoplasmen, Bakterien, Parasiten, tierische Zellen, Proteine oder Peptide die hydrophobe Regionen aufweisen mit einem oder mehreren Lösungsmitteln gemischt werden, um einen Komplex zwischen den Proteinen oder Peptiden und dem Lösungsmittel zu bilden, wonach die Proteine oder Peptide vom Lösungsmittel in Gegenwart einer Glycosidlösung getrennt werden, die ein oder mehrere Glycoside mit hydrophoben und hydrophilen Regionen in einer Konzentration aufweist, die wenigstens der kritischen mizellularen Konzentration entspricht, oder alternativ durch Abtrennung der geladenen monomeren antigenischen Proteine oder Peptide vom Lösungsmittel und ihre direkte Überstellung in die Glycosidlösung, wobei ein Proteinkomplex mit Glycosid geformt wird, der isoliert und gereinigt wird, wonach das Trägermolekül dadurch mit Molekülen verbunden wird, die aus Peptiden, Proteinen, Kohlenhydraten, Lipoproteinen, Glycolipiden oder Biotin gewählt werden, daß eine Kupplung zwischen Funktionsgruppen in diesen Molekülen, die an den Träger zu binden sind und Funktionsgruppen in den Trägermolekülen hergestellt wird.

7. Verfahren nach Anspruch 6, dadurch gekennzeichnet, daß etwa 1 mg, vorzugsweise 250 - 500 µg des anzukuppelnden Moleküls mit etwa 1 mg (als Proteingehalt gemessen) des Trägermoleküls durch Kupplung zwischen den Funktionsgruppen zur Reaktion gebracht werden.

8. Einen Immunstimmulanz bildende Zusammensetzung, dadurch gekennzeichnet, daß sie als aktive Substanz wenigstens einen immunogenischen Komplex nach einem der Ansprüche 1 bis 6 gegebenenfalls in Mischung mit pharmazeutisch annehmbaren Additiven enthält.

9. Impfstoff, dadurch gekennzeichnet, daß er wenigstens einen immunogenischen Komplex nach einem der Ansprüche 1 bis 6 als aktive Substanz enthält.

10. Reagenz, dadurch gekennzeichnet, daß es wenigstens einen immunogenischen Komplex nach einem der Ansprüche 1 bis 5 enthält.

## Patentansprüche für folgenden Vertragsstaat: AT

1. Verfahren zur Herstellung eines immunogenischen Komplexes, dadurch gekennzeichnet, daß ein Trägermolekül durch Mischung von Viren, Mykoplasmen, Bakterien, Parasiten, tierischen Zellen, Proteinen oder Peptiden mit hydrophoben Regionen mit einem oder mehreren Lösungsmitteln hergestellt wird, um einen Komplex zwischen Proteinen oder Peptiden und Lösungsmittel zu bilden, wonach die Proteine oder Peptide vom Lösungsmittel in Gegenwart einer Glycosidlösung getrennt werden, die ein oder mehrere Glycoside, welche hydrophobe und hydrophile Regionen aufweisen, enthält und in einer Konzentration vorliegt, die wenigstens der kritischen mizellularen Konzentration entspricht oder alternativ die Proteine oder Peptide vom Lösungsmittel getrennt und direkt in die Glycosidlösung überstellt werden, wodurch ein Proteinkomplex mit Glycosid geformt wird, wonach das Trägermolekül mit einem oder mehreren Molekülen verbunden wird, die aus Peptiden, Proteinen, Kohlenwasserstoffen, Lipoproteinen, Glycolipiden oder Biotin ausgewählt sind, wobei funktionelle Verbindungsgruppen in den an den Träger gebundenen Molekülen und funktionelle Gruppen im Trägermolekül gekuppelt werden.

2. Verfahren nach Anspruch 1, dadurch gekennzeichnet, daß die an den Träger gekuppelten Moleküle aus Peptiden, die 1 - 40, insbesondere 10 - 25 Aminosäuren aufweisen, ausgewählt werden, wobei dann, wenn die Anzahl der Aminosäuren 10 beträgt, die Peptide an aliphatische Ketten gekuppelt werden können, die 2 - 12 Kohlenstoffatome und 6 - 26 OH-Gruppen aufweisen oder an Peptide, die 1 - 20 Aminosäuren, vorzugsweise hydrophile Aminosäuren aufweisen, über Funktionsgruppen, die insbesondere Aminosäurensequenzen aufweisen, welche repräsentativ für Malaria, Polio, insbesondere 277 - 300 sind, Peptide, die vom Hepatitis-B-Virus erhalten werden, insbesondere Peptide in den Bereichen 22 - 74, 110 - 156 und insbesondere Peptide, welche die Aminosäurensequenzen 144 - 145 aufweisen, Rabies-Virus, insbesondere Peptide in den Bereichen 1 - 50, 290 - 320 und insbesondere im Bereich 100 - 175, Influenza-Virus-Peptide, welche die Aminosäurensequenz 140 - 146, 187 - 196 enthalten oder in der Region Cys 52 - Cys 278 oder 207 - 220 ausgewählt sind und Maul- und Klauenseuche-Virus, insbesondere die Maul- und Klauenseuche Virus-Peptide 141 - 160, 144 - 160, 146 - 154, 144 - 150, 142 - 158, Wachstumfaktoren für T-Zellen, vorzugsweise Peptide, welche die Aminosäurensequenz 79 - 92, 139 - 153 C, 111 - 125 und 18 - 32 C aufweisen, Peptide, die vom Epstein Barr-Virus erhalten werden, insbesondere die Sequenzen A: Aps, Val, Gly, Gly, Lys. Lys, His, Gln, Lev, Asp, Cys, Leu, Leu; B: His, His, Ala, Glu, Asn, Gln, Asn, Pro, Cys, Leu, Leu; C: Ala, Trp, Pro, Asn, Asn, Thr, Glu, Thr, Asp, Phe, Lys, Cys, Leu, Leu; antigenische Determinatoren von HTLV 1, 2 oder 3 Virus-Peptiden in Blutgruppensubstanzen; Peptide, Polypeptide und größere Proteine, insbesondere Steroidhormone, Peptidhormone und Prostaglandinhormone, wie Tyrotropin, Adenocorticotropin, luteinisierende Hormone, Gonadotropin freisetzende Hormone, follikelanregende Hormone, Prclactin, Wachstumshormone, Oxytocin, Vasopressin, Paratyroidea Hormone, Calcitonin, Insulin, Glucagon und Enzyme, wie Lysosym und Peroxidase, Kohlehydrate und kohlehydrathältige Strukturen, wie Lipopoly-Saccharide, Polysaccharide von gekapselten Mikroorganismen, wie E. Coli, insbesondere K-Antigene 1 - 13, Haemophilus Influenza, Meningococcus, den Oligosaccharidanteil von Glycoproteinen in Blutgruppensubstanzen in Gangliosiden, wie GM1 und Glyomganglioside.

3. Verfahren nach Anspruch 1 oder 2, dadurch gekennzeichnet, daß der Träger dadurch erhalten wird, daß die Membranproteine von umhüllten Viren, insbesondere Ortomyxoviridae, Paramyxoviridae, Retroviridae, Rabto-Viridae, Togaviridae, Herpesviridae und Hepatitis-B-Virus, Membranproteine von Toxoplasma, nicht umhüllten Picornaviridae, Parvoviridae und Reoviridae ausgewählt werden und das Glycosid aus Saponinen z. B. als Glycosidextrakt aus beispielsweise Quillaja Saponaria Molina, Aesculus Hippocastanum oder Gyphphilla Struthim, vorzugsweise DQ, Quil A, Ascein, Sapoalbin ausgewählt wird.

4.  Verfahren nach einem der Ansprüche 1 bis 3, dadurch gekennzeichnet, daß der Träger dadurch erhalten wird, daß die Proteine und Peptide von hydrophoben Membranproteinen oder Nicht-Membranproteinen und nicht hydrophoben Proteinen von Viren, Mykoplasmen, Bakterien, Parasiten und tierischen Zellen, welche nicht hydrophoben Proteine durch Ankupplung hydrophober Gruppen an sie hydrophob gemacht werden, die aus aliphatischen Gruppen kleinen hydrophobischen Peptiden und anderen hydrophoben Strukturen gewählt werden, beispielsweise Steroide, wie Gallensäure und Cholesterinderivate, amphiphatische Proteine oder Peptide, die unzugängliche hydrophobe Gruppen enthalten, welche durch milde Denaturisierung zugänglich wurden oder aus auf synthetischem Wege gewonnenen bzw. durch Hydrind-DNA-Technik erzeugten Proteinen oder Peptiden ausgewählt werden.

5.  Verfahren nach den Ansprüchen 1 bis 4, dadurch gekennzeichnet, daß der Träger dadurch erhalten wird, daß Viren, Mykoplasmen, Bakterien, Parasiten, tierische Zellen oder hydrophobe Peptide oder Proteine mit dem Lösungsmittel gemischt werden, das aus einem ionischen, nicht-ionischen, zwitterionischen oder Gallussäure aufweisenden Detergent, Alkoholen, kleinen amphiphatischen Molekülen, wasserlöslichen Peptiden oder Proteinen oder Mischungen davon in gepufferter möglicherweise salziger Lösung ausgewählt wird, die Mischung oben auf eine Lösung abgelegt wird, die Lösungsmittel enthält und die ihrerseits über einem Gradienten liegt, der Glycosid enthält und bei wenigstens 100 000 g zentrifugiert wird, die proteinhältige Fraktion isoliert und gegen eine Pufferlösung dialysiert wird oder die Mikroorganismen, tierischen Zellen, Proteine oder Peptide nachdem sie mit dem Lösungsmittel in gepufferter salziger Lösung gemischt wurden, mit Glycosid reagiert und gegen einen Puffer vorzugsweise Amoniumacetatpuffer, dialysiert oder direkt auf einem Gradienten abgelegt und bei wenigstens 100 000 g zentrifugiert werden, wonach die proteinhältige Oberfraktion gesammelt, mit Glycosid reagiert und gegen einen Puffer, vorzugsweise Amoniumacetat dialysiert wird oder daß die Mischung aus Mikroorganismen, tierischen Zellen, Proteinen oder Peptiden und Lösungsmittel im Puffer oder die proteinhältige Oberfraktion, welche erhalten wird, wenn die Mischung aus Mikroorganismen, tierischen Zellen, Proteinen oder Peptiden und Lösungsmittel in gepufferter salziger Lösung durch einen Gradienten zentrifugiert wird, durch Elektrophorese oder auf chromatographischem Wege vom Lösungsmittel getrennt und in einer Lösung gesammelt wird, welche die Glycoside enthält, wonach der erhaltene Proteinkomplex gegebenenfalls z. B. durch Lyophilisierung, Vakuumdialyse oder Ultrazentrifugierung konzentriert oder durch Gradientenzentrifugierung weiter gereinigt wird.

6.  Verfahren nach Anspruch 5, dadurch gekennzeichnet, daß näherungsweise 1 mg, vorzugsweise 250 - 500 $\mu$g, der anzukuppelnden Moleküle mit näherungsweise 1 mg (als Proteingehalt gemessen) der Trägermoleküle unter Kupplung zwischen den Funktionsgruppen zur Reaktion gebracht wird.

7.  Verfahren zur Herstellung einer als Immunstimmulanz dienenden Zusammensetzung, dadurch gekennzeichnet, daß wenigstens ein immunogenischer Komplex nach einem der Ansprüche 1 bis 6 hergestellt und wahlweise mit pharmazeutisch annehmbaren Additiven gemischt wird.

8.  Verfahren zur Herstellung eines Impfstoffes, dadurch gekennzeichnet, daß wenigstens ein immunogenischer Komplex nach einem der Ansprüche 1 bis 6 hergestellt wird.

9.  Reagenz, dadurch gekennzeichnet, daß er wenistens einen immunogenischen Komplex nach einem der Ansprüche 1 bis 6 enthält.

FIG.1

FIG. 2

FIG.3

FIG.4

FIG.
5A

FIG.
5B